(19) Europäisches Patentamt

European Patent Office

Office européen des brevets

(11) **EP 4 679 442 A1**

(12) **EUROPEAN PATENT APPLICATION**

(43) Date of publication:
14.01.2026  Bulletin 2026/03

(21) Application number: 25187914.4

(22) Date of filing: 07.07.2025

(51) International Patent Classification (IPC):
**G16H 20/17** (2018.01)    **G16H 40/63** (2018.01)
**G16H 50/20** (2018.01)    **G16H 50/70** (2018.01)

(52) Cooperative Patent Classification (CPC):
**G16H 20/17; G16H 40/63; G16H 50/20;
G16H 50/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority:  08.07.2024  US 202463668620 P

(71) Applicant: **Insulet Corporation
Acton, MA 01720 (US)**

(72) Inventors:
• **ZHENG, Yibin
53029 Hartland (US)**
• **LEE, Joon Bok
01720 Acton (US)**
• **SALAVATI, Saeed
77478 Sugar Land (US)**
• **O'CONNOR, Jason
01720 Acton (US)**
• **MEYER, Anthony
28205 Charlotte (US)**

(74) Representative: **Peterreins Schley
Patent- und Rechtsanwälte PartG mbB
Hermann-Sack-Straße 3
80331 München (DE)**

(54) **METHODS TO MODIFY BEHAVIORS TO MITIGATE IMPACT OF PREDICTION INACCURACIES FOR LATER TIME WINDOWS IN AMD SYSTEMS**

(57)    Exemplary embodiments relate to automated medicament delivery (AMD) devices. Such devices may measure the level of an analyte and deliver medicament with the intent of maintaining the analyte at a target level or in a target range. The described methods and apparatuses apply a model to evaluate an effect of proposed future medicament doses on predicted analyte levels. The proposed future medicament doses and predicted analyte levels may be supplied to a cost function to select which of the proposed future medicament dose schedules results in optimal control of the predicted analyte levels. The cost function may apply a weighting scheme that weighs predictions in the near- and/or medium-term future more than longer-term predictions.

FIG. 10

**Description**

CROSS-REFERENCE TO RELATED APPLICATIONS

This application claims priority to and the benefit of U.S. Provisional Application No. 63/668,620, filed July 8, 2024, the entirety of which is incorporated herein by reference.

BACKGROUND

Blood glucose control is the process by which the body maintains a stable level of glucose in the bloodstream, which is crucial for providing energy to the body's cells. The main hormone responsible for blood glucose control is insulin, which is produced by the pancreas.

In people with diabetes, blood glucose control is impaired because the body either doesn't produce enough insulin (Type 1 diabetes) or the body's cells are resistant to insulin (Type 2 diabetes). This leads to high blood glucose levels, which can cause damage to organs and tissues over time.

Managing blood glucose levels is an essential part of diabetes management. This may involve regular monitoring of analyte (such as blood glucose or ketone) levels, medication, dietary changes, and exercise. By carefully managing blood glucose levels, people with diabetes can reduce the risk of complications and lead healthy, active lives.

In order to manage their diabetes, diabetics may use one or more medicaments, such as insulin, glucagon, tirzepatide, glucagon-like peptide (GLP-1), glucose-dependent insulinotropic polypeptide (GIP), a combination of insulin and GLP-1 and/or GIP, and others. Although some examples are described in connection with management of blood glucose, exemplary devices may be suitable for use with other types of medicaments, such as fertility drugs and white blood cell stimulating drugs.

Automated medicament delivery (AMD) systems are designed to improve the glycemic control of people with diabetes. Algorithms within AMD systems often utilize internal models to predict future states of the system to be controlled - for example, the user's predicted blood glucose levels in the future. The algorithm creates predicted trajectories of the glucose concentrations of each user of the AMD system. These predictions and trajectories are generally based on population-average models. Consequently, these predictions become less reliable the further into the future the predictions are made, since individual users are likely to have different characteristics than the average member of the model population. The resulting model-system mismatch is compounded over the prediction horizon. Thus, an AMD system that utilizes the model predicted trajectories may be subject to sub-optimal insulin delivery recommendations due to these inaccuracies.

Furthermore, AMD systems typically utilize a predicted trajectory of analyte and medicament values to assess the future state of its users and adjust its current medicament delivery recommendations. However, in cases where the user's analyte values vary rapidly, the AMD system's predictions may be inaccurate, leading to sub-optimal medicament delivery recommendations. Further, in cases where the user's analyte value is consistently exhibiting a trend, such as a downward trajectory, a limited horizon of an AMD system's predictions may lead to insufficiently responsive medicament delivery recommendations.

BRIEF SUMMARY

In one aspect, a computer-implemented method includes accessing a model of analyte-medicament dynamics associated with a user of an automated medicament delivery device, generating two or more sets of proposed medicament doses representing medicament doses to be delivered to the user over a period of time defined by a prediction horizon, for each set of the proposed medicament doses, using the model to calculate a set of predicted analyte values corresponding to the proposed medicament doses, applying a cost function to each set of the proposed medicament doses and corresponding set of predicted analyte values, where the cost function weighs values that represent earlier doses in the sets to a greater degree than values that represent later doses in the sets that are closer to the prediction horizon, selecting one of the two or more sets of proposed medicament doses based on an output of the cost function, and transmitting a control signal configured to cause the automated medicament delivery device to deliver the medicament in an amount defined by the selected set of proposed medicament doses.

The weighing may be performed in various ways. For example, the weighing may apply weights that are set to predetermined fixed values.

In other embodiments, the weighing may apply a set of weights having a first predetermined fixed value until a threshold cycle is reached, and after the threshold cycle is reached each weight applies a second predetermined fixed value.

In another example, the weighing may apply weights that decrease exponentially to the prediction horizon.

In yet another example, the weighing may apply weights that vary with a value of the current predicted cycle being evaluated.

Furthermore, the weighing may apply weights that are calculated according to a dynamic formulation based on a difference between a predicted trajectory in a previous cycle and a measured analyte value in the previous cycle.

These weighing schemes may be applied separately, or may be mixed-and-matched (within a single application of the cost function, and/or across multiple iterations). The weighing may apply one or more weights, and the weights may be updated at predetermined intervals. The model may also or alternatively be updated based on a prediction accuracy at predetermined intervals.

The prediction horizon may be varied, for example to decrease the prediction horizon as variability in the user's analyte values increases.

The techniques described above may be performed separately or in any combination to achieve synergistic effects. Embodiments further include a non-transitory computer-readable medium storing instructions that, when executed by one or more processors, cause the processors to perform any of the methods as described above. Embodiments further include an automated medicament delivery system including one or more processors, and a non-transitory computer-readable medium storing instructions that, when executed the one or more processors, cause the processors to perform the method of any of the above-described methods.

Other technical features may be readily apparent to one skilled in the art from the following figures, descriptions, and claims.

BRIEF DESCRIPTION OF THE SEVERAL VIEWS OF THE DRAWINGS

To easily identify the discussion of any particular element or act, the most significant digit or digits in a reference number refer to the figure number in which that element is first introduced.

FIG. 1 depicts an exemplary medicament delivery system 100 in accordance with one embodiment.
FIG. 2A depicts a system that integrates an automated insulin delivery device and a continuous glucose monitor in accordance with one embodiment.
FIG. 2B depicts the system of FIG. 2A in greater detail.
FIG. 3A depicts a standalone continuous glucose monitor in accordance with one embodiment.
FIG. 3B depicts the monitor of FIG. 3A in more detail.
FIG. 3C depicts the monitor of FIG. 3A in more detail.
FIG. 4 is a flow chart that provides an overview of a system initialization and operation procedure for a medicament delivery system 100 in accordance with one embodiment.
FIG. 5 depicts the component detection process of FIG. 4 in more detail.
FIG. 6 depicts the initialization process of FIG. 4 in more detail.
FIG. 7A depicts the delivery process of FIG. 4 in more detail
FIG. 7B depicts the event detection process of FIG. 4 in more detail
FIG. 7C depicts the notification process of FIG. 4 in more detail.
FIG. 8 depicts the short-term adaptation process of FIG. 4 in more detail.
FIG. 9 depicts the long-term adaptation process of FIG. 4 in more detail.
FIG. 10 illustrates exemplary logic in accordance with one embodiment.

DETAILED DESCRIPTION

In typical AMD systems, algorithms may utilize a cost function that weighs excursions of predicted glucose and medicament trajectories against target glucose concentrations across a wide range of possible predictions. In this context, an excursion refers to a deviation from a target value. For example, a glucose excursion may represent an amount by which a measured blood-glucose level (e.g., as measured by a continuous glucose monitor) differs from a user's target blood glucose level. A medicament excursion, such as an insulin excursion, may represent an amount by which a delivery of the medicament differs from a target amount or target rate.

The algorithm may determine that the trajectory with the lowest cost is the optimum solution for recommended medicament delivery. In an AMD measuring blood glucose and delivering insulin as a medicament, this cost function may be represented as follows:

$$J(i) = \sum_{j=1}^{P} Q(G_p(j) - SP(j))^2 + R(I_p(j) - B(j))^2$$

Here, the predicted glucose G(j) and insulin I(j) trajectories are compared against the target glucose concentration SP(j) and basal values B(j), respectively, over j cycles, up to a prediction horizon of P cycles (The prediction horizon refers to the final time point included in the prediction). The relative weighting of the glucose and insulin excursions are represented as Q and R, respectively. However, this representation applies an equal contribution of excursions across all predicted cycles from the 1st predicted cycle to the Pth predicted cycle to the total cost, J.

Exemplary embodiments provide methods to modify the weighting applied to predictions based on the number of control cycles in the future that the prediction is executed, when the predictions are incorporated into the AMD system's determination of proposed medicament deliveries. As discussed in more detail below, the cost function may be modified to apply a variable weight to the analyte (e.g., glucose) and medicament (e.g., insulin) excursions, depending on the length of the predicted cycles. These embodiments work on the assumption that the reliability and accuracy of the predicted trajectories may reduced for cycles further into the predictions.

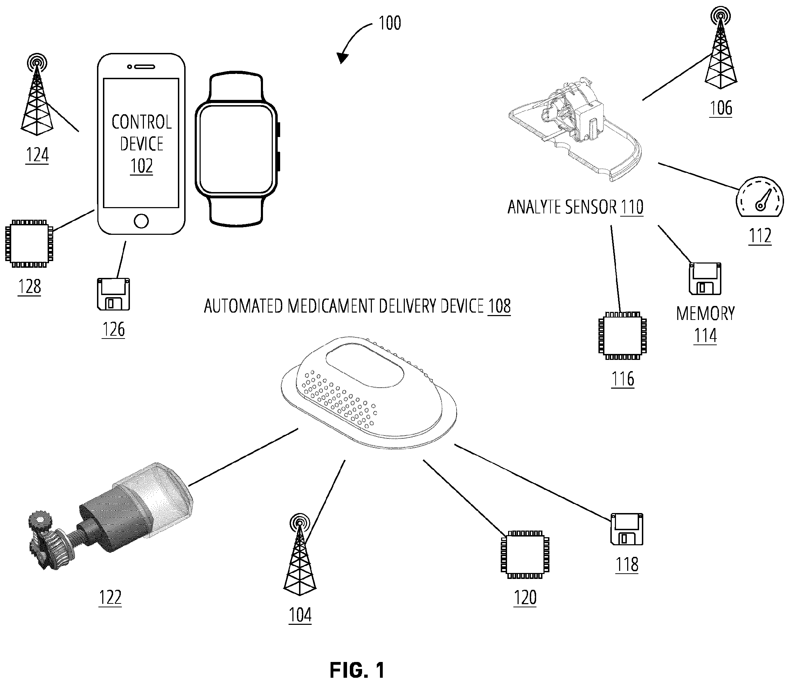

Several different embodiments will be described below. Each provides advantages, and it is contemplated that any embodiment may be employed individually (e.g., in connection with a control algorithm and system as described in connection with FIG. 1 - FIG. 3C), or in any combination to yield synergistic improvements.

*A Note on Data Privacy*

Some embodiments described herein make use of training data or metrics that may include information voluntarily provided by one or more users. In such embodiments, data privacy may be protected in a number of ways.

For example, the user may be required to opt in to any data collection before user data is collected or used. The user may also be provided with the opportunity to opt out of any data collection. Before opting in to data collection, the user may be provided with a description of the ways in which the data will be used, how long the data will be retained, and the safeguards that are in place to protect the data from disclosure.

Any information identifying the user from which the data was collected may be purged or disassociated from the data. In the event that any identifying information needs to be retained (e.g., to meet regulatory requirements), the user may be informed of the collection of the identifying information, the uses that will be made of the identifying information, and the amount of time that the identifying information will be retained. Information specifically identifying the user may be removed and may be replaced with, for example, a generic identification number or other non-specific form of identification.

Once collected, the data may be stored in a secure data storage location that includes safeguards to prevent unauthorized access to the data. The data may be stored in an encrypted format. Identifying information and/or non-identifying information may be purged from the data storage after a predetermined period of time.

Although particular privacy protection techniques are described herein for purposes of illustration, one of ordinary skill in the art will recognize that privacy protected in other manners as well. Further details regarding data privacy are discussed below in the section describing network embodiments.

Assuming a user's privacy conditions are met, exemplary embodiments may be deployed in a wide variety of messaging systems, including messaging in a social network or on a mobile device (e.g., through a messaging client application or via short message service), among other possibilities. An overview of exemplary logic and processes for engaging in synchronous video conversation in a messaging system is next provided.

Exemplary Embodiments

As an aid to understanding, a series of examples will first be presented before detailed descriptions of the underlying implementations are described. It is noted that these examples are intended to be illustrative only and that the present invention is not limited to the embodiments shown.

Reference is now made to the drawings, wherein like reference numerals are used to refer to like elements throughout. In the following description, for purposes of explanation, numerous specific details are set forth in order to provide a thorough understanding thereof. However, the novel embodiments can be practiced without these specific details. In other instances, well known structures and devices are shown in block diagram form in order to facilitate a description thereof. The intention is to cover all modifications, equivalents, and alternatives consistent with the claimed subject matter.

Next, a basic medicament delivery system 100 will be described. As shown in FIG. 1, the medicament delivery system 100 may include an automated medicament delivery device 108, an analyte sensor 110, and a control device 102.

An automated medicament delivery device 108 is a small device that delivers a medicament, such as insulin to the body in a controlled and continuous manner. An automated medicament delivery device 108 allows for more precise and flexible medicament delivery compared to injections, as the user can adjust their basal rate and bolus doses to match their individual medicament needs. It can also provide greater convenience and discretion in daily life.

An exemplary automated medicament delivery device 108 may include a medicament pump 122 with a reservoir that holds the insulin and a plunger inserted into the reservoir. Other exemplary embodiments may be plunger-less. Reciprocating pumps and pumps using a pre-filled cartridge are also contemplated. Embodiments are not limited to these types of delivery mechanisms; any suitable delivery mechanism may be employed.

In the depicted type of pump, medicament is filled into the reservoir of the pump, either directly with a syringe or through a special filling device. In some automated medicament delivery devices 108, the reservoir is provided to the user already filled with medicament. The plunger can be withdrawn from the reservoir to allow the reservoir to be filled, or driven into the reservoir to dispense the medicament through a needle. A small motor or other actuator may drive the plunger forward or backwards (or the plunger may be driven under fluid pressure and/or an action of the user), for instance by turning a lead screw that moves a nut attached to the plunger.

Computing hardware and/or software may control the delivery of medicament. For example, a processor 120 may execute a control algorithm, such as the model predictive control (MPC) algorithm described below. The control algorithm may be embodied as instructions stored in a memory 118, which may be a non-transitory computer-readable medium such as flash memory, a hard drive or solid state drive, removable media, etc. The control algorithm may be stored and run on the pump itself, or on another device that communicates with the pump.

The control algorithm may perform several functions, such as calculating basal rates and bolus doses and delivering such doses. The basal rate is the amount of medicament that is delivered to address the body's normal metabolic needs outside of meals to keep the blood glucose levels stable, or in a euglycemic range. A basal rate may be set based on a user's individual medicament needs. Around the time the user is eating (e.g., when the user is about to eat), they can use the pump to deliver a bolus dose of medicament. A bolus dose is an amount of medicament that is delivered all at once to address consumption of foods such as carbohydrates. In an exemplary hybrid closed-loop or other automated medicament delivery device 108, the user inputs the amount of carbohydrates consumed or intended to be consumed and the algorithm determines the amount of medicament needed based at least on the carbohydrate amount and the current, historical, and/or predicted blood glucose level.

The control algorithm may also generate alerts when a problem arises. For instance, the automated medicament delivery device 108 can alert the user to a condition such as a low battery, an occlusion in the fluid line of the of the automated medicament delivery device 108, or if the user forgets to take a bolus dose.

The status of the automated medicament delivery device 108 and any generated alerts may be displayed, either on a small screen on the automated medicament delivery device 108 itself or on a display of a separate control device 102. The displayed information can include the current basal rate, medicament on board, current glucose level, and/or other important information. The control device 102 may also allow a user to manually enter their blood glucose as measured by a fingerstick glucose meter. Alternatively, the control device 102 may display blood glucose levels as measured with an analyte sensor 110.

The automated medicament delivery device 108 can send information to, or receive information from, the control device 102 and/or analyte sensor 110 using a transmitter/receiver 104. The transmitter/receiver 104 may enable wireless communication; for instance, it may be a BLUETOOTH(R) or BLUETOOTH Low Energy (LE) radio, or a similar type of device. The control device 102 may include a corresponding transmitter/receiver 124, as well as a memory 126 and a processor 128.

An exemplary analyte sensor 110 is a small device that tracks the level of an analyte, such as glucose or ketones, in interstitial fluid (fluid between the body's cells) continuously throughout the day and night. The analyte sensor 110 may provide data to the automated medicament delivery device 108 to allow the automated medicament delivery device 108 to adjust the amount of insulin being delivered. An analyte sensor 110 such as a continuous glucose monitor provides a more complete picture of glucose trends than traditional fingerstick glucose monitoring, as it provides continuous glucose data and alerts. It can help people with diabetes to manage their glucose levels more effectively and improve their overall health and quality of life.

The analyte sensor 110 may include a transmitter/receiver 106 similar to the transmitter/receiver 104 of the automated medicament delivery device 108. The analyte sensor 110 may also include a memory 114 and a processor 116.

The analyte sensor 110 further includes a sensor 112, such as a glucose sensor or a ketone sensor, for example. The sensor 112 is inserted just under the skin, usually on the abdomen, arm, or thigh. The sensor 112 may have a small filament that is inserted into the interstitial fluid and measures the level of certain chemicals in the user's blood.

For example, a glucose sensor is a device used to measure the concentration of glucose in a person's blood or interstitial fluid. There are several types of glucose sensors, but most work by using an enzyme called glucose oxidase to catalyze the reaction between glucose and oxygen.

When glucose oxidase reacts with glucose, it produces gluconic acid and hydrogen peroxide. The amount of hydrogen peroxide produced is proportional to the amount of glucose present. The glucose sensor measures the amount of hydrogen peroxide and uses it to calculate the concentration of glucose in the blood.

A sensor 112 of this type typically consists of one or more small electrodes that are coated with the enzyme glucose oxidase. The electrodes are inserted under the skin and are connected to a small transmitter that sends the glucose readings to a receiver in the analyte sensor 110.

Another type of sensor 112 is a ketone sensor. A ketone sensor works by measuring the concentration of ketones, which are produced when the body breaks down fat for energy.

A ketone sensor is similar to a glucose sensor in that it uses an enzyme (e.g., beta-hydroxybutyrate dehydrogenase) to detect ketone levels. This enzyme catalyzes the reaction between beta-hydroxybutyrate, a type of ketone, and a coenzyme called NAD+. The reaction produces NADH and acetoacetate, another type of ketone. The ketone sensor measures the amount of NADH produced by the reaction and uses it to calculate the concentration of beta-hydroxybutyrate in the blood. This gives an indication of the level of ketosis in the body.

The sensor 112 is typically good for several days' or weeks' use, and the user can monitor their glucose and/or ketone levels in real-time using the control device 102. The sensor 112 measures the user's glucose and/or ketone level every few minutes and sends this data to the transmitter/receiver 106. The transmitter/receiver 106 may then send this data wirelessly to the transmitter/receiver 104 of the automated medicament delivery device 108 and/or to the transmitter/receiver 124 of the control device 102. In some embodiments where the automated medicament delivery device 108 and analyte sensor 110 are integrated or co-located, a physical (e.g., wired) connection may be provided to allow the automated medicament delivery device 108 and analyte sensor 110 to communicate.

The control device 102 may collect and analyze the glucose and/or ketone data, displaying it as a graph or number. It also provides alerts if the glucose level and/or ketone level goes too high or too low, helping the user to take appropriate action to manage their glucose and/or ketone levels. The data can further be used to identify trends and patterns in glucose and/or ketone levels over time, helping the user to make informed decisions about diet, exercise, medication, and other factors that affect their glucose and/or ketone levels.

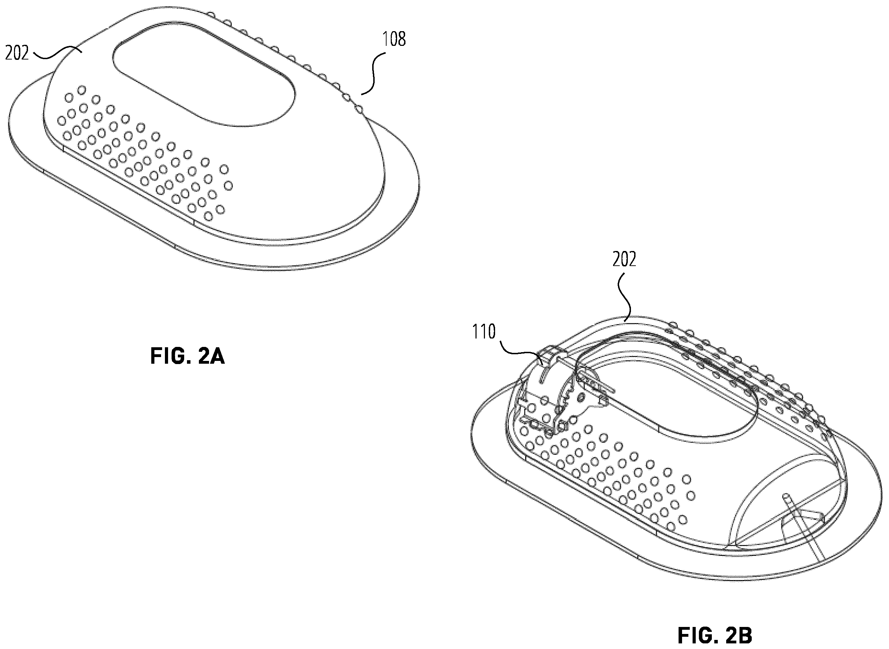

The automated medicament delivery device 108 and analyte sensor 110 may be integrated together in a single device, as shown in FIG. 2A and FIG. 2B. In this example, the device includes at least one housing 202 that encompasses both the automated medicament delivery device 108 and the analyte sensor 110.

The automated medicament delivery devices 108 and analyte sensor 110 may not have the same lifespans. For example, the reservoir or cartridge of the automated medicament delivery device 108 may only store enough insulin for a few days, while the sensor 112 of the analyte sensor 110 may remain useful for several more days.

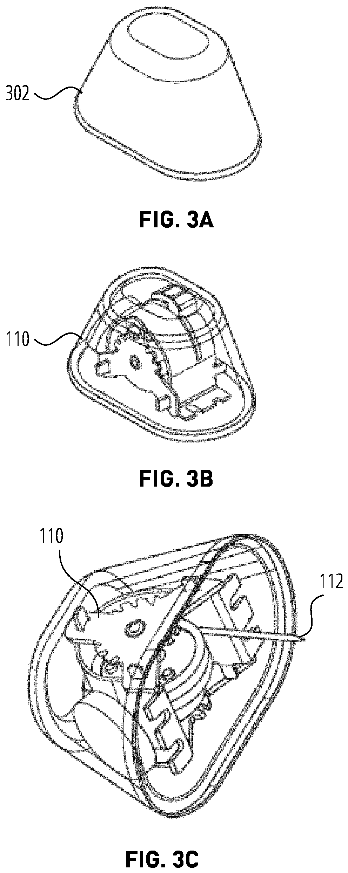

Accordingly, in some embodiments the analyte sensor 110 may be provided in its own analyte sensor housing 302, as shown in FIG. 3A - FIG. 3C. In such embodiments, separate housings may encompass the automated medicament delivery device 108 and the analyte sensor housing 302, and such housings may be positioned adjacent to each other, e.g., on a tray or a cradle or another housing, thereby allowing the automated medicament delivery device 108 to be separately replaced.

Next, a glucose control algorithm will be described. The glucose control algorithm described below represents a basic algorithm that may be further modified using the techniques described in the remainder of the application.

Control Architecture

The control algorithm may employ a modular design in which core functionality is separated from dependent functionality. Dependent functionality includes functionality that is implementation-specific to the current environment. This includes software abstraction for the analyte sensor 110. The dependent functionality also includes software services which interface with implementation-specific features that affect inputs or outputs to the algorithm, such as the Activity Mode (see below).

An interface may be responsible for managing algorithm initialization and upload of insulin history to a history buffer, managing the algorithm's state and data variables, and maintaining cycle-to-cycle data needed by the algorithm. For this purpose, an analyte sensor interface may retrieve data such as blood glucose and/or ketone measurements from the analyte sensor 110.

The interface may also send commands to the automated medicament delivery device 108 to deliver micro-bolus outputs as calculated by the algorithm. This transmission of data may occur wirelessly or over a bus of the automated medicament delivery device 108. The transmission may occur at regular intervals (e.g., every 1 minute, every 5 minutes, etc.).

The algorithm's core functionality may include a set of procedures that take an estimated glucose value (EGV) as input and compute a recommended insulin dose (in the example of an automated insulin delivery device; other devices employing different sensors and/or medicaments may also be used and the algorithm adjusted accordingly). These layers also include a wide range of safety constraints and edge case handling routines to ensure robust and safe behavior of the algorithm.

System Parameters

The control algorithm relies on a wide range of parameters and features to operate effectively to calculate its recommended medicament dose. Many parameters remain fixed or are derived from user adjustable inputs. These variable inputs that impact algorithm parameters may include glycemic setpoint, total daily medicament, whether Activity Mode is activated, and user-requested meal and correction boluses. In some embodiments, total daily medicament may be input once, for the first day of medicament delivery, or initially calculated from the user's basal profile. In other embodiments, total daily medicament maybe derived from other parameters without requiring user input.

Glycemic Setpoint

A glycemic setpoint is a blood glucose target between 90 and 150 mg/dL that the algorithm uses as a baseline. The glycemic setpoint is a concept used to describe the blood glucose level that the body strives to maintain during normal activity. It is similar to the idea of a "set point" for body weight or temperature, which the body works to maintain within a certain range.

In healthy individuals without diabetes, the body's glycemic level is typically around 80-120 mg/dL (4.4-6.7 mmol/L) when fasting and up to 180 mg/dL (7.8 mmol/L) or higher after a meal. The body maintains euglycemia through a complex interplay of hormones, including insulin and glucagon, which help to regulate glucose production and uptake in the liver and muscles.

In people with diabetes, the glycemic level can be altered due to a range of factors, including insulin resistance, impaired insulin production, and lifestyle factors such as diet and exercise. Imbalanced factors can lead to chronically high blood glucose levels (hyperglycemia) or low blood glucose levels (hypoglycemia), both of which can have negative health consequences.

Managing blood glucose levels through medication, dietary changes, and lifestyle modifications can help to bring blood glucose levels closer to the body's natural glycemic setpoint or euglycemic range, reducing the risk of complications and improving overall health.

The control algorithm supports the use of multiple target glucose values set by the user, e.g., 90-150 mg/dL in 10 mg/dL increments.

Total Daily Medicament

Total daily medicament (TDM) refers to the amount of insulin or another medicament a person with diabetes needs to take each day to maintain normal blood glucose levels. This includes both basal insulin, which provides a steady background level of insulin, and bolus insulin, which is taken before meals to cover the rise in blood glucose that occurs after eating.

The total daily medicament dose can vary greatly depending on a person's individual needs, including factors such as body weight, level of physical activity, diet, stress, and insulin sensitivity. For women, it can also vary based on period of a menstrual cycle or stage of a pregnancy. It is typically calculated based on a person's insulin-to-carbohydrate ratio (ICR) and insulin sensitivity factor (ISF), which are determined through blood glucose monitoring.

In the control algorithm, TDM may be user-adjustable for the first 48 hours of use and afterwards may be dependent on the user's medicament delivery history.

Activity Mode Activation

Activity Mode activation refers to whether the user activated or did not activate a mode that may be referred to as "Activity Mode." The Activity Mode temporarily changes parameters that are passed to the algorithm to make insulin delivery less aggressive in order to protect against hypoglycemic events, for example, during periods of inactivity. Triggering Activity Mode temporarily sets insulin delivery to a lower value to prevent entering hypoglycemic range during activities which can lower blood glucose level.

User Requested Meal and Correction Boluses

User requested meal and correction boluses refer to the size of a bolus that can be given parallel to algorithm delivery to accompany ingestion of any meals.

System Initialization

Achieving optimal blood glucose control through insulin therapy requires careful monitoring and adjustment of insulin doses based on individual needs and changing circumstances. People with diabetes who use insulin may work with a healthcare team to develop an individualized insulin regimen that meets their needs and helps them achieve their blood glucose goals. During algorithm initialization, the user's health care provider may assist the user to program basal rates, glucose targets and bolus calculator settings, although in some embodiments it may not be necessary to receive assistance from a health care provider.

Ongoing Blood Glucose Control

The exemplary control algorithm described below is a model predictive control (MPC) algorithm. Model Predictive Control (MPC) is an advanced computational control approach that uses a mathematical model of a system to make predictions of future system behavior and optimize control actions.

The MPC algorithm uses a dynamic model of the system (in this case, the system may represent the insulin-glucose dynamics of the user) and an optimization algorithm to predict the future behavior of the system and find an optimal control action. For instance, the algorithm may predict the user's future blood glucose in response to a proposed set of insulin doses.

The algorithm optimizes the control input by minimizing a cost function over a finite time horizon, subject to constraints on the system variables and control input. A cost function, also known as an objective function or loss function, is a mathematical function that quantifies the error or discrepancy between the predicted output of a model and the actual or desired output. In exemplary embodiments, the cost function may assess the cost of predicted glucose deviations (from a setpoint) and insulin delivery deviations (from the user's typical basal rate); several iterations may be run through and the one with the lowest cost may be chosen. One purpose of a cost function is to measure how well a model is performing and to guide the learning or optimization process towards finding the optimal set of parameters. By minimizing or maximizing the cost function, the model can adjust its parameters to achieve the desired outcome. Examples of common types of cost functions include (but are not limited to) Mean Squared Error (MSE), Binary Cross-Entropy, Categorical Cross-Entropy, Hinge Loss, and Kullback-Leibler Divergence.

The control action is applied over the finite time horizon, after which the optimization is repeated using the updated system state. This process is repeated iteratively, resulting in a sequence of optimal control actions that are applied to the system over time.

MPC has many advantages, including the ability to handle complex multivariable systems with constraints, the ability to incorporate disturbances and uncertainties into the control strategy, and the ability to handle non-linear dynamics.

The model within the medicament delivery system 100 may predict the evolution of analyte concentrations, such as glucose concentrations, over a prediction horizon consisting of a certain number (e.g., 6, 12, 24, etc.) time-steps of relatively short (e.g., 1, 3, 5, 10, etc. minutes) duration. This results in a predicted glucose over a prediction horizon (e.g., a 30-, 60-, 120-, etc. minute horizon). The model takes in estimated glucose values (EGV) from the analyte sensor 110 through Bluetooth Low Energy (BLE) and calculates small doses of insulin accordingly for the automated medicament delivery device 108 to deliver.

In obtaining blood glucose measurements, the algorithm may apply rate of change filters. The rate of change filters factor in the real change in analyte sensor values that can occur in the human body over a period of time due to physiology. They may also limit glucose predictions of the system to be no more than a threshold value.

The algorithm may also maintain the analyte sensor 110 state based upon analyte sensor 110 values received to determine the output of the algorithm. It also can provide estimates of missing analyte sensor 110 data handling and analyte sensor predictions. The missing data handling provides logic that controls the automated medicament delivery device 108, allowing the automated medicament delivery device 108 to behave robustly when the analyte sensor 110 is not available due to communication interruptions between the automated medicament delivery device 108 and the analyte sensor 110. In one example, this medicament may be insulin.

The model may penalize both deviations of predicted glucose values from the setpoint as well as deviations of the recommended insulin from a determined insulin basal rate. At each time-step, the algorithm determines an optimal set of insulin doses (which my be referred to as basal doses or micro-boluses) over a control horizon consisting of a certain number of timesteps that minimizes the cost of these two deviations. MPC is based on real-time numerical optimization and thus allows for a large degree of flexibility in achieving the control objective. Based on the optimal control action identified by the algorithm over the time horizon (e.g., an insulin dose that minimizes the cost function over the time horizon), a medicament dosage amount may be determined and a medicament dose command may be transmitted to the medicament pump 122.

At each time-step, the medicament dose command from the algorithm is bounded by multiple safety constraints on medicament delivery, as managed by algorithm constants and safety limits.

These constraints include a dynamic constraint based on a medicament-on-board (MOB) model incorporated into the optimization algorithm. Medicament on board (MOB) refers to the amount of medicament, such as insulin, that has not yet acted in a user's body after their last medicament dose. This is typically measured in units of medicament and is used to help people with diabetes manage their blood sugar levels.

When the user injects insulin or uses an insulin pump, the insulin begins working to lower their blood sugar. However, it takes some time for the insulin to take effect, and even after it does, it continues to work for several hours. This means that there may be a period of time during which the user has both the insulin from their previous dose and the insulin from their current dose working in their body.

Knowing MOB can be helpful for managing blood sugar levels, as it can give an idea of how much insulin is still working in a user's body and how it may be affecting the user's blood sugar. Some automated medicament delivery devices and analyte sensors can estimate MOB automatically based on the amount and timing of medicament doses.

Optimal post-prandial control may require the user to give meal boluses (as is required in conventional pump therapy), but normal operation of the MPC algorithm may compensate for missed meal boluses and mitigate prolonged severe hyperglycemia. During operation, logic may detect rescue carbohydrate ingestion and apply safe upper limits to micro bolus delivery. Rescue carbs handling logic may detect rescue carbohydrate ingestion based on readings from the analyte sensor 110 and/or from other external sensors.

System Changes

The automated medicament delivery device 108 may be used for a certain period of time and then replaced by a new automated medicament delivery device 108. During such a system change, the user's medicament data (including TDM) is saved to a buffer (e.g., stored on the control device 102 and/or a remote server). During every AMD system change, before deactivation of the medicament delivery system 100, the data from the buffer is transferred to an app on the control device 102, which updates its previously saved data, storing new data to be consumed by the MPC Algorithm. Over several medicament delivery system 100 changes, the algorithm adapts to the user's TDM needs.

The algorithm estimates the Medicament on Board (MOB) from automated medicament delivery and provides this to the app on the control device 102. The Algorithm MOB, along with recent boluses recorded by the app, are utilized to calculate total MOB. The total MOB is displayed to the user on the control device 102.

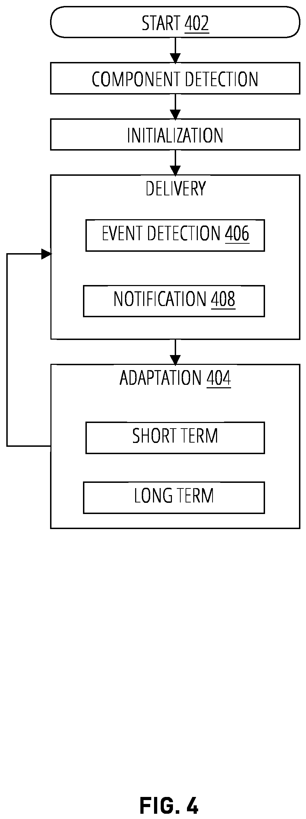

FIG. 4 illustrates an example routine for controlling an automated medicament delivery device 108 with limited or no user input. Although the example routine depicts a particular sequence of operations, the sequence may be altered without departing from the scope of the present disclosure. For example, some of the operations depicted may be performed in parallel or in a different sequence that does not materially affect the function of the routine. In other examples, different components of an example device or system that implements the routine may perform functions at substantially the same time or in a specific sequence.

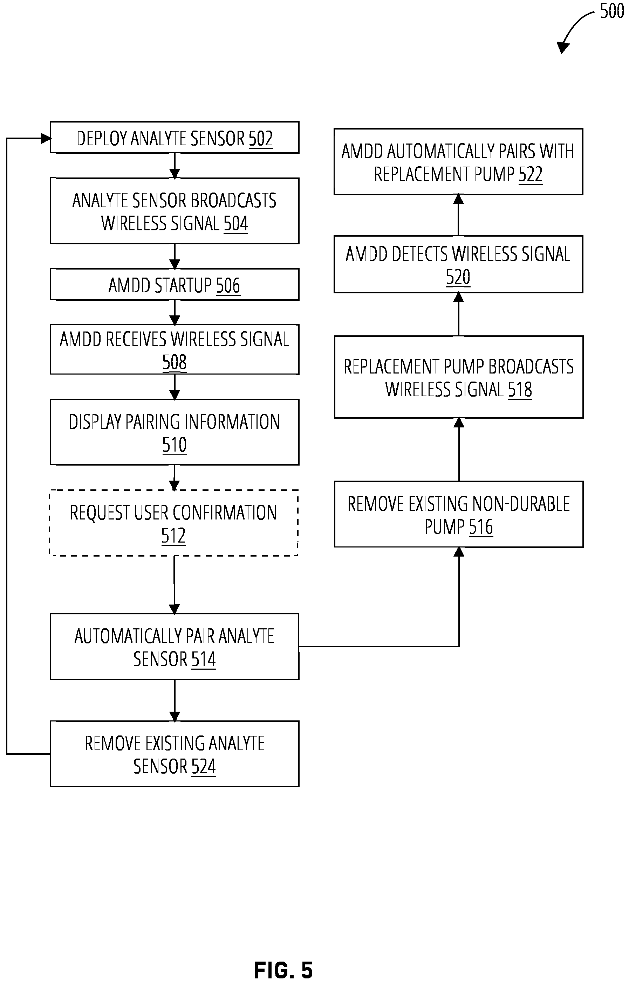

Note that the blocks depicted in FIG. 4 reference general processes that may be performed in connection with the deployment and operation of an AMD. Existing processes are known for some of these steps, although exemplary processes are expressly described for each process in connection with FIG. 5 - FIG. 9. It is contemplated that all of the novel processes may be used in the overall processes, or a mix of old and new processes may be employed. Thus, the present disclosure is not limited to the situation where all of the processes of FIG. 5 - FIG. 9 are used in the appropriate blocks in FIG. 4. Nonetheless, it is contemplated that all of these processes could be used together in an exemplary embodiment.

Processing begins at start block 402. Start block 402 may be triggered, for example, upon an initial startup of a new AMD system. The initial startup may trigger provisioning logic that causes the AMD system to perform a first-time setup procedure.

Processing may then proceed to component detection process 500, where the AMD system performs component detection. As previously discussed, AMD systems typically incorporate an analyte sensor 110 and an automated medicament delivery device 108 including a medicament pump 122 controlled by an AMD algorithm. The user interface that allows the user to assess the status of the system and/or interact with the system may be part of the automated medicament delivery device 108, in case of durable components, or it may be a separate component such as the control device 102.

A simplified, no-setting AMD system may automatically detect an advertising analyte sensor 110 via their Bluetooth signal, and pair with the analyte sensor 110 without requiring any user input. Optionally, the AMD may ask for confirmation that the sensor and AMD system should be paired. Moreover, the automated medicament delivery device 108 may automatically detect a non-durable medicament pump 122 that has been "primed" and in range, without the user needing to enter any identifying parameters. In a similar manner, a new AMD system may automatically establish a connection with an existing and operational AMD system. Security measures, such as physical on-device confirmation display requirements, may be employed. The component detection process is described in more detail in connection with FIG. 5.

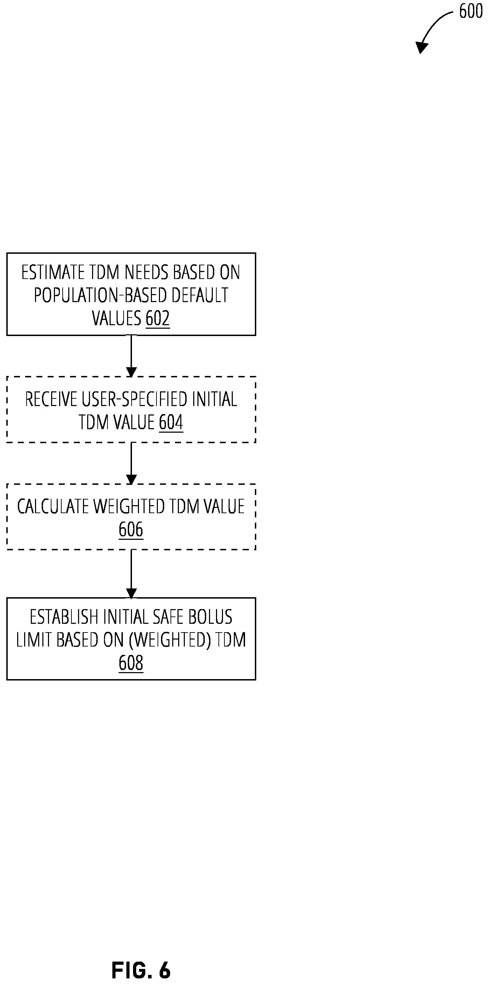

According to some examples, the method may proceed with initialization at initialization process 600. The initialization process 600 sets system parameters used to determine amounts and/or timings of medicament doses to initial values. These values may be derived from a total daily medicament value. The initial total daily medicament value may be determined, wholly or in part, based on population-based default values. A predicted-safe value may be used for the initial total daily medicament value. The predicted-safe value may be based on the population-based default value and the capability of the automated medicament delivery device 108 to compensate for over- or under-delivery of medicament. The initialization process 600 is described in more detail in connection with FIG. 6.

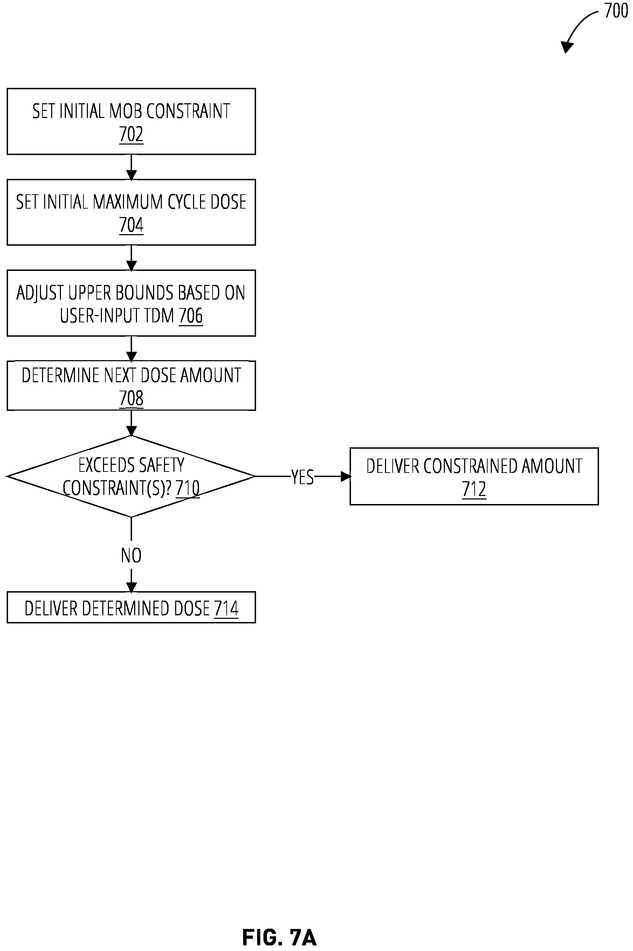

According to some examples, the method includes delivering at delivery process 700. As noted above, the process of delivering medicament to a user may be subject to several safety constraints (e.g., a medicament-on-board constraint, a maximum cycle dose constraint, system upper bounds, etc). Values for the safety constraints may be initially set based on the initial total daily medicament value determined in the initialization process 600. The delivery process is described in more detail in connection with FIG. 7A.

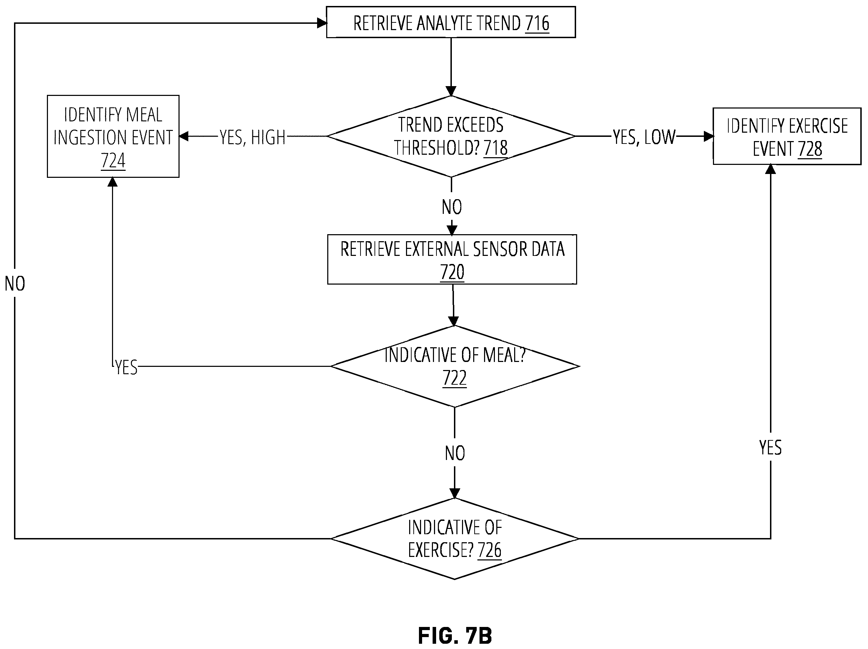

According to some examples, the method includes event detection at event detection process 406. The event detection process may use data available to the automated medicament delivery device 108 and/or control device 102 to determine whether an analyte control event is occurring. For example, in the case of controlling a user's blood glucose, events such as meal ingestion or exercise may affect the ability of the automated medicament delivery device 108 to control the user's blood glucose. Detection of such an event may cause the automated medicament delivery device 108 to enter into a different operating state or mode, or to otherwise adjust medicament delivery parameters. Events may be detected, for example, by reviewing analyte trends from analyte values measured by the analyte sensor 110 and/or by reviewing external sensor data from devices such as the control device 102, a fitness tracker, global positioning device, etc. The event detection process is described in more detail in connection with FIG. 7B.

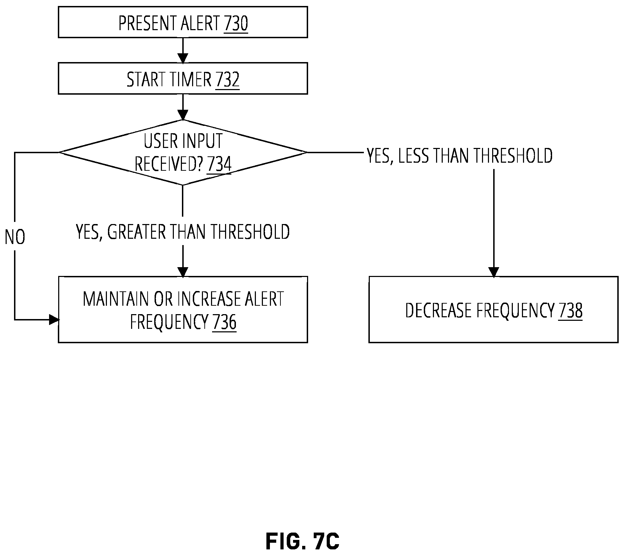

According to some examples, the method includes a notification process at notification process 408. When the user experiences an analyte control event (e.g., an instance of hypo- or hyper-glycemia), a notification may be generated to inform the user of the event. Depending on how the user interacts with the notification, the frequency of notifications may be adjusted. For example, if the user generally acknowledges notifications quickly, the rate or frequency of notifications may be decreased so as to not interfere with the user's daily activities. On the other hand, if the user fails to respond to one or more notifications, the frequency of notifications may be increased, the notification may be escalated (e.g., by adding sound or vibration), and/or the notification may be escalated to third parties such as authorized emergency contacts. The notification process is described in more detail in connection with FIG. 7C.

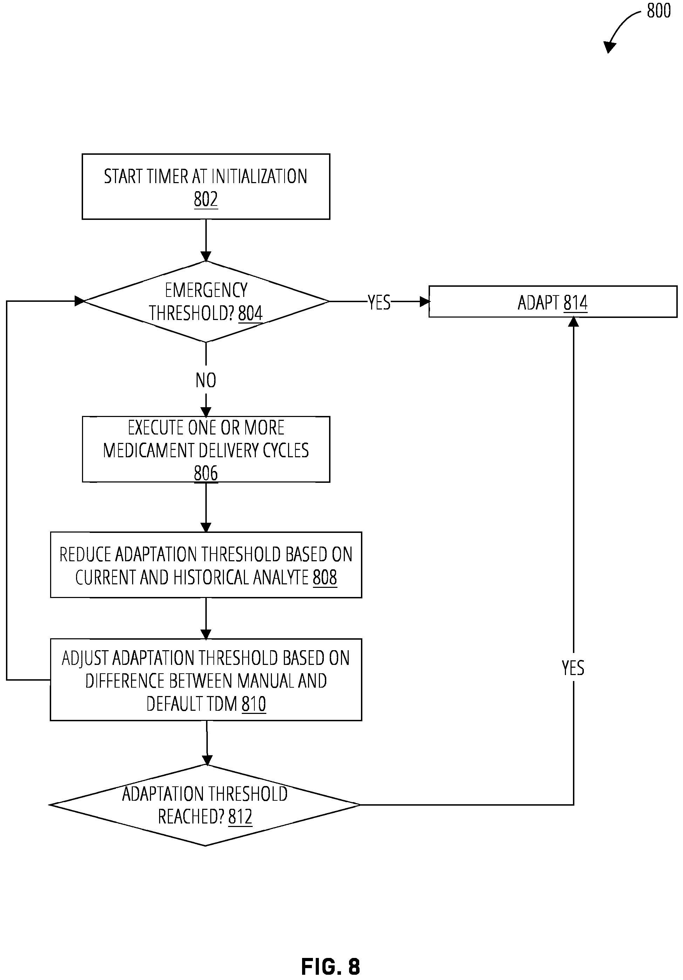

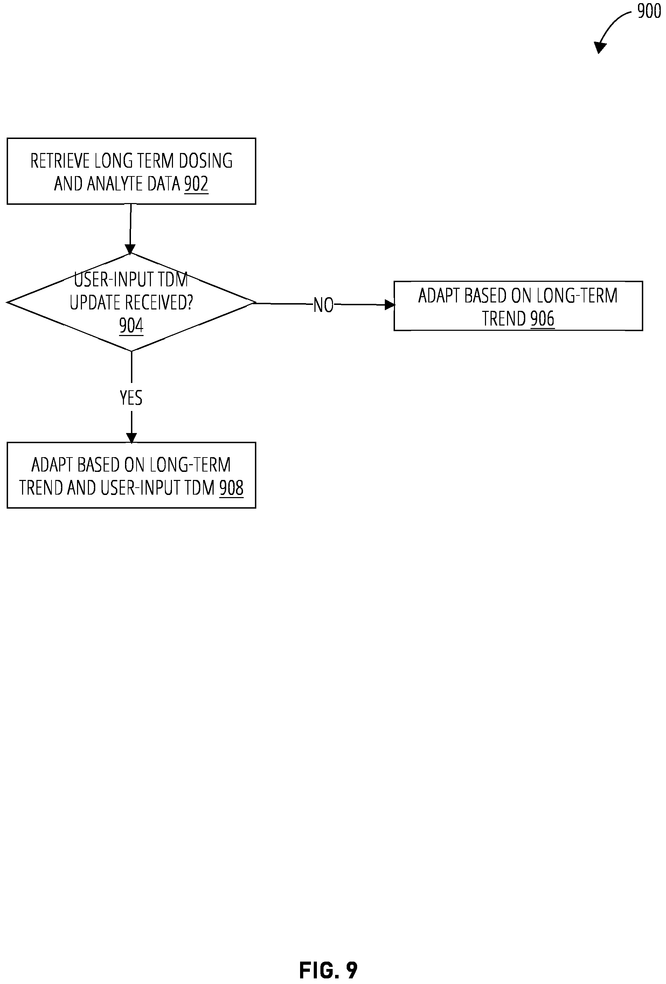

According to some examples, the method includes an adaptation process at block 404. The adaptation process adjusts the initial values for total daily medicament and other parameters over time, as additional analyte measurement data becomes available. The adaptation process may include a short term adaptation process 800 (discussed in more detail in connection with FIG. 8) and a long term adaptation process 900 (discussed in more detail in connection with FIG. 9).

FIG. 5 depicts the component detection process 500 of FIG. 4 in more detail. Although the example component detection process 500 depicts a particular sequence of operations, the sequence may be altered without departing from the scope of the present disclosure. For example, some of the operations depicted may be performed in parallel or in a different sequence that does not materially affect the function of the component detection process 500. In other examples, different components of an example device or system that implements the component detection process 500 may perform functions at substantially the same time or in a specific sequence.

According to some examples, the method includes deploying an analyte sensor 110 at block 502. The analyte sensor 110 may be a standalone device that is deployed separately from an automated medicament delivery device 108. The analyte sensor 110 may be placed on a user's body.

The analyte sensor 110 may be configured to broadcast measurements of an analyte at regular intervals to connected devices (e.g., the automated medicament delivery device 108). In order to connect to these devices, according to some examples, the analyte sensor may broadcast a wireless (e.g., Bluetooth) signal at block 504. The wireless signal may be an initiation of a device-specific handshake procedure.

According to some examples, the method includes automated medicament delivery device 108 startup at block 506. For example, the user may fill the automated medicament delivery device 108 with medicament (or medicament may be pre-loaded in the automated medicament delivery device 108), and then the user may place the automated medicament delivery device 108 on their body. The automated medicament delivery device 108 may detect that it has been placed on the body (e.g., based on a sensor output). Upon detecting its placement on the body, the automated medicament delivery device 108 may begin scanning for a broadcast wireless signal, such as the aforementioned handshake procedure.

According to some examples, the automated medicament delivery device 108 may receive the wireless signal at block 508. At block 510, the automated medicament delivery device 108 may optionally cause pairing information (e.g., a device identifier of the analyte sensor 110 attempting to pair with the automated medicament delivery device 108) on a display of the automated medicament delivery device 108 or the control device 102.

According to some examples, automated medicament delivery device 108 may optionally request user confirmation that the analyte sensor 110 should be paired at block 512. For example, a display of the automated medicament delivery device 108 or the control device 102 may display an indication that the analyte sensor 110 is attempting to pair, and asking that the user accept the pairing (e.g., by tapping an on-screen confirmation button). In some embodiments, the user may confirm the pairing by not canceling the pairing request within a predetermined period of time (e.g., but not tapping an on-screen cancelation button).

According to some examples, analyte sensor 110 may be automatically paired with the automated medicament delivery device 108 without any (further) user interaction at block 514. For example, the automated medicament delivery device 108 and analyte sensor 110 may complete the aforementioned handshake process to establish a communication connection.

In some embodiments, the component detection process 500 may terminate at this stage, or (through block 524) may revert back to block 502 so that the old analyte sensor 110 can be removed and a new analyte sensor 110 can be paired with the automated medicament delivery device 108 with limited or no user interaction.

In other embodiments, the component detection process 500 may also allow the medicament pump 122 of the automated medicament delivery device 108 to be removed and replaced, and for the automated medicament delivery device 108 to establish communication with the new medicament pump 122. To this end, at block 516 an existing non-durable (i.e., replaceable) pump may be removed.

When a new pump is primed (e.g., filled with a medicament and placed in a configuration such that it is capable of dispensing the medicament), then the new pump may begin broadcasting a wireless handshake signal (similar to the one previously described in connection with the analyte sensor 110) at block 518.

The automated medicament delivery device 108 may detect the wireless signal at block 520 and, in a manner similar to blocks 510-514 above, may automatically pair with the replacement pump at block 522.

Although the above described procedure relates to pairing an analyte sensor 110 with an automated medicament delivery device 108, the same technique may be used to pair other devices with the automated medicament delivery device 108 without requiring user input. For example, the automated medicament delivery device 108 may be paired with a heart rate sensor, accelerometer, the control device 102, a wearable fitness or medical tracker, etc.

Once the medicament delivery system 100 is connected across all relevant components, the medicament delivery system 100 may also initiate its insulin deliveries without any user input (or with only limited user input as described below).

Typical medicament delivery systems 100 operate based on a set of parameters, such as the user's analyte (e.g., glucose) control target, total medicament (e.g., insulin) needs, and/or bolus medicament needs. However, a robustly designed medicament delivery system 100 can start medicament delivery without prior user input. This may be achieved by a thorough risk assessment and various constraints to allow the system to deliver a reduced insulin delivery amount, which when paired with analyte feedback will protect users from over-delivery. Previous medicament delivery and/or analyte history from devices that are connected may be used to improve this initialization.

A reasonable, safe assumption of initial parameters, such as basal medicament needs, target analyte concentrations, and general system settings, can be executed to cover a great majority of each user's use patterns. This may be based on typical population metrics, with reasonable statistical analyses of standard deviations and the AMD algorithm's robustness designs.

FIG. 6 illustrates an example initialization process 600 in more detail. Although the example initialization process 600 depicts a particular sequence of operations, the sequence may be altered without departing from the scope of the present disclosure. For example, some of the operations depicted may be performed in parallel or in a different sequence that does not materially affect the function of the initialization process 600. In other examples, different components of an example device or system that implements the initialization process 600 may perform functions at substantially the same time or in a specific sequence.

According to some examples, the method includes estimating total daily medicament needs based on population-based default values at block 602.

The most fundamental clinical parameter utilized by people with insulin-dependent diabetes is total daily medicament, or TDM. This TDM value may be further incorporated into calculations for a wide range of derivative clinical parameters, such as basal insulin profiles, insulin to carbohydrate ratio, correction factor, and others. Consequently, TDM values are available for most, if not all, users who may transition into AMD systems, even if those users do not yet utilize the derivative parameters, such as is the case for multiple daily injection users.

When transitioning to a no- or limited- user-input paradigm, an initial TDM value must be determined that protects against over- or under- delivery of medicament. If too much medicament is provided to a diabetic user, the user may experience hypoglycemia as their blood glucose level drops to dangerous levels. If too little medicament is provided, the user may experience hyperglycemia as their blood glucose level rises to dangerous levels.

One insight useful in setting the initial TDM value is that, when paired with an analyte sensor 110 that retrieves frequent measurements of the user's analyte levels (e.g., blood glucose or ketones), a suitable automated medicament delivery device 108 can make continuous adjustments to the amount of medicament delivered, and can therefore provide robust protection against medicament over- or under delivery (up to a certain degree). The degree to which the automated medicament delivery device 108 and analyte sensor 110 can compensate for an initial over- or under- estimate of TDM can inform the initial selection of the TDM value.

More specifically, AMD systems typically deliver insulin in smaller increments, as an AMD algorithm often incorporates continuous medicament measurements from an analyte sensor 110 such as a continuous glucose monitor (CGM). Therefore, it is feasible for an AMD system to potentially be initialized with a relatively higher insulin delivery rate, and allow the AMD system's feedback mechanisms to reduce delivery as needed. In converse, there is a significantly greater risk in bolus deliveries, particularly manual bolus deliveries, due to the large quantity of any one-time delivery, magnifying the impact of erroneous initializations. Therefore, a safe initialized system may limit the initial delivery over time.

For example, the general population's average total insulin needs may be approximately 40U. A reasonable starting estimate for AMD systems may then be a total insulin value below that amount, assuming the system can adapt rapidly to the user's insulin needs based on analyte outcomes. In one embodiment, this value may be set to 20U, assuming that the AMD system may be able to compensate for reduced insulin need for users with up to 50% less than this value (such as 10U):

$$TDI_{est}(1) = 20U$$

In one example embodiment, an AMD system may initialize its parameters with an assumed safe initial estimate of the user's TDM without any inputs from the user. However, such an AMD system will generally behave in a conservative manner to minimize risk of over delivery of insulin and resulting hypoglycemia. This may be embodied as 1) a low initial estimate of the default TDM value, 2) stronger limitation by the system's safety constraints, and 3) a slow rate of change of the TDM parameter.

Optionally, the user may specify an initial TDM value at block 604. Although next generation AMD systems may be designed to reduce or eliminate the inputs needed for users to both initialize and maintain glucose control outcomes, optionally providing TDM values as inputs may still improve the performance of the AMD systems. Moreover, TDM values may be commonly available for most users who first desire to utilize AMD systems, and may not represent a significant increase in complexity for the use of AMD systems.

Therefore, exemplary embodiments allow users to optionally provide this TDM as an input to the algorithms, allowing the system to incorporate modifications to both its initialization and subsequent behaviors, as needed. This can take a number of forms. The user may have previous data from (e.g.) manual medicament dosage data. Based on this, the user may have an estimate of their TDM values. Alternatively, a physician may estimate the TDM value for the user, and a physician-input TDM may be received by the system.

As a further alternative, the user's physician may develop a full basal profile, which may be input into the system. A basal profile, also known as a basal rate profile, is a term commonly used in the context of insulin therapy for people with diabetes. The basal profile refers to the programmed schedule of insulin delivery that provides a continuous, low level of insulin to the body to meet the basal metabolic needs of the individual throughout the day and night. The basal profile is an important aspect of medicament therapy for people with diabetes, as it helps to maintain consistent blood glucose levels and prevent high or low blood sugar episodes.

In an automated medicament delivery device 108, the basal profile may be set up by the healthcare provider or the patient, and it specifies the amount and timing of medicament delivery at different times of the day. The basal rate is typically adjusted based on individual needs and can vary depending on factors such as physical activity, stress, and mealtime insulin doses. Based on the amounts and timings of medicament delivery in the basal profile, the TDM may be calculated.

Depending on how a manual TDM value is entered, the component detection process 500 may apply weighting to determine a weighted TDM value at block 606. A user-input TDM might be less-trusted than the population-derived initial TDM value, since a user might make a mistake in calculating their TDM. A physician-generated or -input TDM may be more trusted than a user-input TDM, and indeed might be more trusted than the population-derived value since it has been customized to a particular user by a reliable source. A physician-generated basal profile might be the most trusted, to the point of (potentially) overriding the population-derived TDM.

Accordingly, If the user provides an optional TDM input that demonstrates a significantly increased insulin need compared to the low initial estimate, this can be reflected as follows. Note that the below equations are defined with respect to total daily insulin (TDI), assuming that insulin is the medicament. If another medicament is used, the appropriate TDM value for that medicament would be used in said equation. Hence, in the following, while the equations use "TDI", they also refer to "TDM".

The initial TDM estimate utilized by the AMD algorithm may apply a weighted sum of the system's default value, and the user's input TDM parameter. This can be captured as follows:

$$TDI_i = W \bullet TDI_d + (1 - W)TDI_m$$

Given that a user's input TDM (TDM$_M$) will be manually entered, and may thus potentially be non ideal for the user, the weight for these parameters $W$ may be heavily towards the default value TDM$_d$ (e.g., $W > 0.5$, preferably $0.6 < W < 0.95$, or more preferably $0.7 < W < 0.9$). A physician-input TDM might have a different weighting (e.g., $W < 0.5$, preferably 0.05 < W < 0.4, or more preferably 0.1 < W < 0.3). A TDM derived from an input basal profile might be weighed very heavily (e.g., $W < 0.3$, preferably $0 \leq W < 0.2$, or more preferably $0 \leq W < 0.1$)

Alternately or in addition, the system may be validated to be safe for a significant majority of users for two or more different sets of initial default parameters. If the user's input TDM value is below a certain threshold, the initial TDM may remain as the lowest initial parameter, whereas a higher input TDM by the user may allow the system to select a higher, but still safe, initial default parameter. This may be captured as follows:

| TDM category | Final TDM$_i$ | User input TDM |
|---|---|---|
| TDM(default, no input) | 10U | TDM$_m \leq$ 25U |
| TDM-(default, Low) | 20U | 25U <TDM$_m \leq$ 40U |
| TDM(default, Medium) | 30U | 40U < TDM$_m \leq$ 55U |
| TDM(Default, High) | 40U | 55U < TDM$_m$ |

The values listed in each cell of this table are examples only, and the number of categories and each value within the cells can be customized.

According to some examples, the method further includes establishing an initial safe bolus limit based on the (weighted) TDM at block 608.

Specifically, the user's bolus deliveries may be limited to no more than an amount that may be potentially safe for the user. This may be calculated by assuming a risk of "no harm" in over-delivery as equivalent to an additional (e.g.) 0.5x basal delivery for up to 6 hours. This leads to a threshold of 3x basal over 6 hours, or, 0.75x hourly basal in a 90 minute period (peak time of insulin action). Therefore, an erroneous one-time over-delivery may be limited to no more than 0.75x the hourly basal for the lowest insulin need we are attempting to safely compensate for the user - such as 50% of the current estimated TDM that is safe for AMD systems. Therefore, the maximum bolus delivery at any one cycle may be set as:

$$b_{max}\left(i\right) \;=\; 0.75 \;\cdot\; \frac{0.5 TDM_i(i)}{48} \;=\; 0.0078 \cdot TDM_i\left(i\right)$$

The delivery of the user's basal dose (which may be given in a series of background micro-doses over the course of a day) may then be constrained by $b_{max}$ such that the amount of basal dosage delivered in a given cycle (i) does not exceed $b_{max}$.

In this way, the initial estimate of the TDM may be calculated and an initial constraint limiting the amount of an acceptable bolus dose over a predetermined time window may be determined. Next, the medicament delivery system 100 may derive other constraints from the initial TDM estimate and may begin to deliver medicament based on the TDM and constraints.

An AMD algorithm also operates under a set of constraints to limit medicament delivery. However, these constraints, when initialized, may be modified to improve analyte outcomes without incurring additional risk to users.

FIG. 7A illustrates an example delivery process 700. Although the example delivery process 700 depicts a particular sequence of operations, the sequence may be altered without departing from the scope of the present disclosure. For example, some of the operations depicted may be performed in parallel or in a different sequence that does not materially affect the function of the delivery process 700. In other examples, different components of an example device or system that implements the delivery process 700 may perform functions at substantially the same time or in a specific sequence.

According to some examples, the method includes setting an initial medicament on board (MOB) constraint at block 702. The medicament-on-board constraint limits a maximum delivery of medicament to no greater than the amount required to bring the user to the target analyte concentrations.

However, the calculation for the amount needed for glucose reduction may be modified to account for the uncertain TDM. One example of an insulin-on-board constraint that accounts for uncertain TDM is shown below:

$$U_{max,IOB}(i) = \frac{\frac{CGM(i) - \max(120, target(i))}{1600}}{0.5 * TDI_{est}(i)} - IOB(i)$$

Here, the heuristic rule of thumb for correction factor may be reduced by 50%, and the glucose target may be fixed at 120 mg/dL or higher to reduce risk of over-delivery. Other values may be used, i.e., a reduction value between 30% to 70%, or a glucose target between 100 mg/dL to 150 mg/dL.

A further constraint is the maximum cycle dose. According to some examples, the method includes setting initial maximum cycle dose at block 704.

At block 704, the total allowed automated insulin delivery constraints at any one cycle may also be modified to a fixed value or lower, with similar principles to the maximum basal delivery. Specifically, at any 3 hour cycle, the system may limit maximum total delivery basal to the threshold no greater than an overdose Low, or no more than an additional 1x basal over 6 hours, or 1x additional delivery per hour. Other values than the aforementioned, and those used in the following equation may be used. This may be translated to a fixed value based on a reduced estimate of the system's current TDM estimate, as follows:

$$U_{max,integral,3\ hours}(i) = 3 \cdot \frac{0.5TDI_{est}(i)}{48} = \frac{1.5TDI_{est}(i)}{48}$$

Yet another constraint is the upper bound, or maximum delivery, possible for the AMD system. According to some examples, the method includes adjusting the upper bound based on the user-input TDM at block 706.

In this scenario, the user's TDM inputs can be incorporated to modify the system's initial, conservative safety constraints. At a high level, these safety constraints may indicate a limitation to the upper bound, or maximum delivery by the AMD system. A modification of this initial conservative constraint may be affected as follows:

$$UB_i = \min\left(UB_{max}, \left(UB_d + 0.25 \bullet \frac{TDI_m - TDI_d}{TDI_d}\right)\right)$$

Here, the upper bound may be increased by the proportion between the user's manually entered TDM versus the default TDM value, with the direct incorporation scaled by a tunable parameter (0.25 in this example).

Alternatively, as above, there may be one or more thresholds between the default conservative safety constraints versus a higher, but still conservative safety constraint demonstrated to be safe for the users, which may be selected if the user's TDM is higher than a threshold - similar to the table shown above in connection with the initialization process 600).

The above-described constraints may be adjusted over time as more data from the analyte sensor 110 becomes available. As the medicament delivery system 100 learns how the user responds to different doses of insulin, the constraints may be relaxed over time. In the absence of sufficient data, the constraints may be more limiting.

According to some examples, the method includes determining next dose amount at block 708. Using the MPC algorithm described above, the system attempts to select a dosage that minimizes a cost function. This generates an initial calculated value for the next dose. At decision block 710, this initial calculated value is compared to the above-described constraints (e.g., $U_{max,\ MOB}$, $U_{max,\ integral,\ 3\ hours}$, $UB_i$). If the initial calculated value does not exceed any of the constraints, then at block 714 the automated medicament delivery device 108 may generate and transmit a control signal configured to cause the medicament pump 122 to deliver an amount of medicament corresponding to the initial calculated value. If the initial calculated value exceeds one or more of the constraints, then the lowest constrained value is selected and at block 712 the automated medicament delivery device 108 may generate and transmit a control signal configured to cause the medicament pump 122 to deliver an amount of medicament corresponding to the constrained value.

Another key set of inputs typically utilized by AMD systems is the incorporation of announcement of disturbances to analyte (e.g., glucose) control that cannot be found by analyte concentration values - such as meal ingestion, exercise, and/or other factors. The detection of these events can be simplified through AMD system improvements on existing inputs, and/or automatic incorporation of other data sources to detect events without user input.

FIG. 7B illustrates an example event detection process 406. Although the example delivery process 700 depicts a particular sequence of operations, the sequence may be altered without departing from the scope of the present disclosure. For example, some of the operations depicted may be performed in parallel or in a different sequence that does not materially affect the function of the delivery process 700. In other examples, different components of an example device or system that implements the delivery process 700 may perform functions at substantially the same time or in a specific sequence.

According to some examples, the method includes retrieving an analyte trend at block 716. The analyte trend may be a series of analyte measurements as returned by the analyte sensor 110 over a given period of time (e.g., the past 30 minutes) or in certain contexts (e.g., following meal ingestion or exercise).

The AMD system may review different characteristics in analyte and medicament delivery trends to detect analyte control events. For instance, a rapid rise in glucose concentrations may be characteristic of a meal ingestion. A rapid reduction in glucose may be characteristic of an exercise. Models may be created based on typical responses of each user to a given medicament delivery value - and significant deviations from the trajectories calculated by the model can be utilized to detect a wide range of system behaviors.

According to some examples, the method includes evaluating recent analyte trends (e.g., over a predetermined recent time frame, such as 5, 10, 15, 30, 60, ... minutes) at decision block 718. The trends may be compared to the aforementioned model to determined if the trend is indicative of (e.g.) meal ingestion or exercise. In some embodiments, the predetermine recent time frame is between 5 minutes to 120 minutes, more specifically 10 minutes to 60 minutes long.

For instance, if the trend indicates that the user's analyte levels have increased above a certain high threshold (or the current trend indicates that the high threshold will be exceeded within a predetermined period of time), this may indicate that the user is ingesting or has recently ingested a meal. Accordingly, at block 724 the system may flag a meal ingestion event. The detection of this event may have a number of consequences. The recent analyte trend may be used to further train the model to better identify meal ingestion events in the future. Alternatively or in addition, the medicament delivery system 100 may shift into a meal ingestion mode that causes an increased amount of medicament to be delivered, or may adjust system parameters or constraints to allow for additional medicament delivery over a predetermined horizon (e.g., based on the amount of time that a meal is predicted to result in increased analyte levels).

On the other hand, if the trend indicates that the user's analyte levels have decreased below a certain low threshold (or the current trend indicates that the low threshold will be met within a predetermined period of time), this may indicate that the user is exercising or has recently exercised. Accordingly, at block 728, the system may flag an exercise event. The detection of this event may have a number of consequences. The recent analyte trend may be used to further train the model to better identify exercise events in the future. Alternatively or in addition, the medicament delivery system 100 may shift into an exercise mode that causes a reduced amount of medicament to be delivered, or may adjust system parameters or constraints to allow for reduced medicament delivery over a predetermined horizon (e.g., based on the amount of time that the exercise is predicted to result in decreased analyte levels).

Furthermore, the AMD system may automatically incorporate a wide range of sensors without user input that are already present in the typical environment. This may allow the system to enter different states or adjust system parameters when an analyte trend has not yet been detected (or when a trend is not recognized by the models). For instance, a user's smartphone typically includes a wide range of sensors, such as accelerometry, GPS, and others. A user's smart watch may incorporate even wide range of health related sensors, such as heart rate, skin temperature, or others. An AMD system may incorporate each of these sensors into its environment to automatically detect events as they are available, dynamically improving its event detection without user inputs. For instance, if the accelerometer indicates a pattern typical of exercise, the AMD system may incorporate this data into its insulin deliveries. If the GPS system indicates that the user is within range of a restaurant, the system may increase its accuracy of detecting a meal disturbance.

Therefore, according to some examples, the method includes retrieving external sensor data at block 720. The external sensor data may include data from sensors on the control device 102 and/or other sensors, such as a smartwatch, fitness tracker, GPS, etc.

At decision block 722, the method may determine if the sensor data is indicative of the user eating or preparing to eat a meal. For example, if the GPS data indicates that the user is near a restaurant, the system may increase sensitivity to the detection of a meal ingestion event (e.g., relaxing parameters of the meal ingestion model so that the meal ingestion model is more likely to report a positive result). If historical data indicates that the user is present near a restaurant at which they have eaten before, or at which they have eaten frequently, the meal ingestion sensitivity may be further increased. Other examples of sensor data that might be indicative of a meal ingestion event may include the user entering meal parameters or checking a calorie count on a fitness device or app, the user turning on their kitchen lights at a time at which they have traditionally eaten a meal, etc. This may result in the detection of a meal ingestion event (block 724).

At decision block 726, the method may determine if the sensor data is indicative of the exercising or preparing to exercise. For example, if the GPS data indicates that the user is near a gym, park, pool, walking path, etc., the system may increase sensitivity to the detection of an exercise event (e.g., relaxing parameters of the exercise model so that the exercise model is more likely to report a positive result). If historical data indicates that the user is present near a gym, pool, park, or other location at which they have worked out before, or at which they have worked out frequently, the exercise sensitivity may be further increased. Other examples of sensor data that might be indicative of an exercise event may include the user accessing a workout program on a fitness device or app, an accelerometer or heart rate monitor indicating that the user has increased their activity level (especially at a time that the user has traditionally exercised), etc. This may result in the detection of an exercise event (block 728).

The AMD system may also adapt its non-glucose related system settings based on user interactions with the system. For instance, an AMD system, by necessity of ensuring user safety, will require certain alerts and/or alarms to be present for system failures. Depending on whether a user is prompt in responding to such alerts, the AMD system may reduce its frequency of alerts and alarms automatically to minimize disturbances to the user's daily lives without impacting the user's safety.

Alternately, if the user often does not interact with an available alert/alarm, the AMD system may increase its frequency of notifications to the users to ensure the user is made aware of potential risks to their diabetes care. For example, a user's blood glucose level might be low, but the user may be in a loud environment and cannot hear the alert. Alternatively, the user might be asleep. If the rate of interaction drops below a certain threshold, the system may increase the frequency of alerts or alarms.

FIG. 7C illustrates an example notification process 40. Although the example notification process 408 depicts a particular sequence of operations, the sequence may be altered without departing from the scope of the present disclosure. For example, some of the operations depicted may be performed in parallel or in a different sequence that does not materially affect the function of the notification process 408. In other examples, different components of an example device or system that implements the notification process 408 may perform functions at substantially the same time or in a specific sequence.

According to some examples, the method includes presenting alert at block 730. The alert may be indicative of, and sent in response to the detection of, an analyte control event (e.g., the user's blood glucose rising above or dropping below a predetermined threshold) or may be a system alert (e.g., indicating a problem with the medicament delivery system 100).

The system may measure how long it takes for the user to respond to the alert. Accordingly, in some examples, the method includes starting timer at block 732. Starting a timer may refer to actively starting a clock that counts up from zero or down from a predetermined number, or may simply involve noting the time at which the timer was started for later comparison to the current time.

The user may or may not respond to the alert by interacting with the medicament delivery system 100. At decision block 734, the system may determine if user input has been received.

If the user does not respond within a predetermined maximum period of time, or the user does respond but after a predetermined high response time threshold, then processing may proceed to block 736 and the medicament delivery system 100 may maintaining or increase a parameter indicative of alert frequency. An increased alert frequency parameter may decrease the time until an alert is re-issued, or may cause the alert to be issued sooner (e.g., the threshold for detecting the analyte control event may be adjusted so that the user is alerted sooner, such as when their blood glucose level has approached but not yet reached the previous event threshold).

In some embodiments, the system may, a block 736, escalate the alerts or alarms. For example, the system may increase the volume of the alert, or add vibration to the alert. The system may alert additional devices (e.g., if the original alert was presented through a sound/vibration on the automated medicament delivery device 108, an escalated alert may include noise or vibration issued from the control device 102). In further embodiments, if a user does not respond to alerts and the analyte sensor 110 is transmitting signals consistent with a dangerous condition, the system may transmit an alert to the user's designated emergency contact or to a physician or medical care facility.

In further embodiments, a low rate of interactivity might also indicate that the user is not using their pump as normally. In response, the automated medicament delivery device 108 may taper down medicament delivery. For example, if the user is in a car accident or in a hospital, it may not be safe to deliver normal amounts of medicament to the user. In these circumstances, the automated medicament delivery device 108 may reduce and eventually eliminate (or reduce to a minimum safe basal value) the amount of medicament being delivered.

On the other hand, if the user does respond to the notification in a timely manner (less than a low threshold amount of time), then the alert frequency may be decreased at block 738.

The decision at decision block 734 may be made based on historical data. For example, the user's response times over a predetermined time horizon may be averaged, and the alert frequency may be adjusted based on the average. This may prevent the alert frequency from being changed if the user was unable to respond to an alert due to an isolated event (e.g., receiving a phone call) or if an otherwise slow responder happened to respond quickly to a single notification.

Once the TDM and constraints are initialized, they can be rapidly adapted based on data from the analyte sensor 110 to improve user outcomes. As an initial matter, this covers the AMD system's short-term adaptivity, where a conservative medicament delivery at the initiation of AMD system adapts within a short period (such as every 3 or 6 hours) to increase delivery for users with higher medicament needs based on analyte feedback. This change in the system may update the system's clinical parameters, such as basal medicament needs, analyte concentrations, or maximum medicament delivery limits, among others. The adaptivity period may be decreased, so that the algorithm adapts more quickly, under certain circumstances.

FIG. 8 illustrates an example short term adaptation process 800. Although the example short term adaptation process 800 depicts a particular sequence of operations, the sequence may be altered without departing from the scope of the present disclosure. For example, some of the operations depicted may be performed in parallel or in a different sequence that does not materially affect the function of the short term adaptation process 800. In other examples, different components of an example device or system that implements the short term adaptation process 800 may perform functions at substantially the same time or in a specific sequence.

According to some examples, the method includes starting timer at initialization at block 802. Starting a timer may refer to actively starting a clock that counts up from zero or down from a predetermined number, or may simply involve noting the time at which the timer was started for later comparison to the current time. The medicament delivery system 100 may execute adaptation logic when the timer reaches the end of an adaptation threshold. The timer may be set to adapt the parameters, for example, every 3, 4, 5, or 6 hours. This value may be accelerated as described below.

There is, however, a situation in which it might be useful to adapt immediately. If the user's analyte levels drop below or rise above predetermined danger thresholds (e.g., a blood glucose concentration below 40 mg/dL or above 400 mg/dL, respectively, indicated by a "YES" at decision block 804), then processing may proceed to block 814 and the medicament delivery system 100 may adapt immediately.

According to some examples, the method includes executing one or more medicament delivery cycles at block 806. The medicament delivery system 100 may execute one or more treatment cycles before re-evaluating whether to adapt (or may re-evaluate after predetermined periods of time, such as every 5, 10, 15, 30, etc. minutes).

According to some examples, the method includes reducing the adaptation threshold based on current and historical analyte at block 808. In an AMD system with no (or limited) inputs, the AMD system must initialize conservatively, leading to potentially safe delivery for users with low insulin needs, but sub-optimal behavior for users with high insulin needs. Instead, the rate of adaptivity for the AMD system may be modified based on the current and historical glucose. In one embodiment, this may be captured as the number of remaining hours until the system executes its next adaptivity routine:

$$H_{adapt}(i) = P_{adapt}\left(1 + N_{adapt}\right)$$
$$- \frac{1}{12}\left(i + N_{CGM_{i-12P_{adapt}...i}>180} + 2\left(N_{CGM_{i-12P_{adapt}...i}\leq 55} + N_{CGM_{i-12P_{adapt}...i}\geq 300}\right)\right)$$

Here, the period of adaptivity, in hours, may be decreased by 5 minutes every control cycle, and further reduced by the number of cycles of hyperglycemia and hypoglycemia - with the cycles of hyperglycemia above 300 mg/dL and hypoglycemia below 55 mg/dL resulting in accelerating the adaptivity rate by 3 times the standard rate. Other threshold values for hyperglycemia, i.e., 280 mg/dL, or hypoglycemia, i.e., 65 mg/dL and for accelerating the adaptivity rate (i.e., by 2 times to 4 times) may be used. These values are exemplary only, and both the thresholds and the acceleration rate may be adjusted as needed.

Alternately or in addition, the adaptation threshold can also be modified based on the number of times the user interacts with the system, as indicated by the number of user's boluses, in a similar manner:

$$H_{adapt}(i) = P_{adapt}\left(1 + N_{adapt}\right) - \frac{1}{12}\left(i + 3N_{bolus_{i-12P_{adapt}...i}}\right)$$

Here, each user bolus since the previous adaptation may result in an acceleration of the adaptivity rate by 4 times the standard rate. This principle can be extended to the emergency threshold determination at decision block 804: if the user interacts with the system multiple times within a predetermined time period (e.g., requests 10 or more bolus doses within a relatively short period of time), the system may detect an emergency condition and immediately adapt.

According to some examples, the method further includes adjusting the adaptation threshold based on a difference between a manual and default TDM at block 810.

The initial TDM estimate utilized by the AMD algorithm may be adapted to the user's estimates once the system gains sufficient history of the user's insulin delivery. The duration of time before the system determines there is sufficient insulin history may be modified by the user's initial TDM inputs, if the system's ongoing insulin delivery history matches the user's TDM inputs. This may be captured (for example) as follows:

$$T_a\left(j\right) = T_{a,D} - 12 \bullet \max\left(0, \left(1 - \min\left(1, \frac{j}{288}\right) \bullet \frac{\left|TDI_m - \frac{\sum_{k=1}^{j} I(k)}{j} \bullet 288\right|}{TDI_m}\right)\right)$$

Here, the default time that the system may wait before the system begins adapting its TDM input, $T_{a,D}$ may be modified by the difference between the TDM manually entered by the user versus the actual insulin delivery history by the user, capped to at most (e.g.) 12 hours and scaling until the system has at least (e.g.) 1 day, or 288 cycles, of data. These values are exemplary only, and may be tuned as needed based on the application.

At decision block 812, the system determines if the adaptation threshold (potentially accelerated as noted above) has been reached. If so, processing proceeds to block 814 and the system initiates adaptation logic. If not, processing returns to block 804 and the system again determines whether the user's analyte levels have breached an emergency threshold (thus triggering immediate adaptation).

According to some examples, the method includes adapting parameters of the automated medicament delivery algorithm at block 814. The parameters may include, for example, basal medicament needs, analyte concentrations, medicament strength, maximum medicament delivery limits, or a total daily medicament value, among other possibilities. The parameters may be adapted based on the analyte measurement history and trends. For example, if the analyte measurement history indicates that the user is not receiving a sufficient amount of medicament to keep analyte levels within a target range, the basal medicament dosage may be increased (and/or the maximum medicament delivery limits may be increased, and/or the total daily medicament value may be increased and/or the medicament strength may be increased). In a similar manner, if the user is receiving too much medicament, these values may be lowered.

Adaptation also covers the AMD system's long term adaptivity, as changes in the user's physiology, such as puberty, pregnancy, and/or illness, that result in persistent, long term changes required for the AMD system's parameters. FIG. 9 depicts the long-term adaptation process of FIG. 6 in more detail.

At block 902, the system may receive long-term dosing information and analyte sensor data that, together, describe how the user's response to their medicament regimen has changed over a predetermined period of time (e.g., 30 days, 40 days, 50 days, 60 days, 3 months, 6 months, one year, etc.). This long term change may be calculated using changes in the trends of the available data for the user, such as previous analyte and/or medicament delivery history.

In some cases, the user may also desire to modify the system's current behavior by entering a new TDM estimate at decision block 904, after the AMD system has started utilizing its own insulin delivery history for calculation of the user's insulin needs. If the user has not provided an updated TDM estimate, then processing may proceed to block 906 and the system may adapt system parameters (e.g., the same system parameters as may be adapted in the short term adaptation process 800) to address long term trends.

If the user did provide an updated TDM estimate at decision block 904, the user's input TDM parameter may also be incorporated as an additional parameter in the ongoing adaptation of the TDM value. In one embodiment, this may be considered as follows:

$$TDI_k = \left(1 - \frac{D_k}{3}\right)TDI_{k-1} + \frac{D_k}{3}TDI_{hist,k}$$

Here, TDM$_k$ indicates the actual TDM that would be utilized by the AMD system at the kth iteration of adaptation, and D$_k$ represents the duration of time of "new" history, in days, that is valid for the historical TDM used in this adaptation, or TDM$_{hist,k}$. The user's manual TDM input may be incorporated into this historical TDM, as follows:

$$TDI_{hist} = (1 - W_a)I_{tot,k} + W_a \frac{\sum_{q=1}^{N} TDI_{m,q}}{N}$$

Where the historical TDM is generated by weighted sum of the average daily total insulin needs in the kth cycle and an average of the user's input TDM values. The weighted sum may weigh more heavily towards the historical estimate the longer the system has had the user's insulin history, with potential values being calculated as follows:

$$W_a = \max\left(\min\left(0.3, 1 - \frac{D_k}{60}\right), 0.05\right)$$

Here, the maximum weight provided to the user's input TDM is 0.3, the minimum weight is 0.05, and the weight may scale linearly between approximately 42 days and 57 days of system use - with the minimum weight applied once more than 57 days of system use.

Each of the terms in the aforementioned equations may be adjusted as needed based on the application.

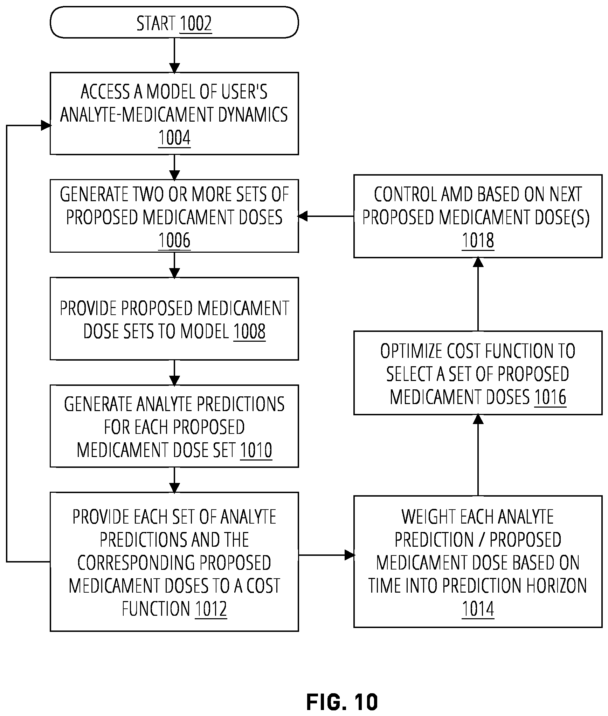

Exemplary logic for carrying evaluating a cost function by weighting near-term predictions more than longer-term predictions is depicted in FIG. 10 - FIG. 10C. The logic may be stored as instructions on a non-transitory computer-readable medium. The instructions may be configured to be performed on a processor of a computing device, such as the automated medicament delivery device 108 or the control device 102 of FIG. 1. Although the logic is depicted in terms of certain actions performed in a particular order, it will be understood that exemplary embodiments may employ more or fewer actions than depicted, in the same or a different order if the changes do not materially affect the function of the routine. Furthermore, the actions may be performed serially or in parallel, as appropriate, and may be split between multiple devices or performed on a single device.

Examples will be provided alongside the description of the logic. The examples are meant to be illustrative only; exemplary embodiments are not limited to the specific calculations performed in the example.

According to some examples, processing may begin at start block 1002. Start block 1002 may be performed, for example, upon system startup. In some embodiments, the system may wait until a certain amount of prediction, analyte measurement, and/or medicament delivery history has been obtained. Thus, processing may proceed after a predetermined period of time has elapsed since device startup.

According to some examples, the method includes accessing a model of user's analyte-medicament dynamics at block 1004. The term "model of user's analyte-medicament dynamics" as used herein may refer to a model that characterizes the effect of a medicament on a physiological parameter measurable as an analyte, such as the effect of insulin on blood glucose concentration, including pharmacokinetic and/or pharmacodynamic components. Here, the system may access and/or initialize a model that is general to a population or customized to the specific user of the AMD. The model may accept, as an input, one or more proposed doses of medicament and may apply the model dynamics to predict how the user's body will react to the proposed doses of medicament. The model may output a predicted analyte level for the user at a specific time in the future or at multiple times in the future.

The model may be embodied as one or more rules, formulas, or as a machine learning model or algorithm, as a combination of multiple models, or any other type of model that may be appropriate. The model may be trained on population-level data and/or on user-specific data, and may be updated as predictions are made and then actual analyte readings are made by the analyte sensor 110. The model may use weights or parameters that map the input dose(s) to the output prediction(s), and these weights or parameters may be updated over time (e.g., at predetermined intervals such as every prediction cycle, every 30 minutes, once an hour, once per day, once per week, etc.) so that the model's predictions better conform to the actual values measured by the analyte sensor 110. In some embodiments, the length of the predetermined intervals is between about 30 minutes to about one month, more specifically between about 12 hours to one week.

According to some examples, the method includes generating two or more sets of proposed medicament doses at block 1006. Each set of proposed medicament doses may include a number P of doses representing medicament doses to be applied from the beginning to the end of a prediction horizon. A set of proposed medicament doses may be represented as an array or vector of size P. For instance, a medicament vector may be represented as $M_p$, where $M_p(i)$ represents the $i$th medicament dose in the set and $0 < i \leq P$.

Each of the doses in a set may be of the same fixed amount (with different sets using different fixed amounts), and/or a set may change the dosage amounts over time. For example, the dosage amount may be changed based on a history of the user's analyte response to a dose of medicament; if the user's need for medicament typically goes up or down as more medicament is delivered, this may be accounted for by adjusting the doses later in the set up or down. In other embodiments, the doses may vary according to a heuristic or best practice. In one example, one or more predetermined medicament dosage profiles may be applied. These profiles may be based on the user's target basal rate and may vary the dosages up or down from that rate. In still further embodiments, an initial set of doses may be evaluated by the model in block 1008. Those sets that are determined to be the best may be kept and the rest discarded, and variations on the best sets may be generated until the user's analyte response reaches a predetermined threshold or no longer changes by at least a predetermined amount.

According to some examples, the method includes providing proposed medicament dose sets to the model at block 1008. The proposed medicament dose sets (or individual doses within the sets) may each be input into the model. The model may also utilize other parameter values, such as the user's measured or predicted analyte level, time of day at which the dose is being simulated, etc.

Based on the inputs, the model may generate an analyte prediction for each proposed medicament dose at block 1010. The analyte prediction may be an estimate or calculation of the predicted analyte level (e.g., a user's predicted blood glucose level) at a certain time j within the prediction horizon P. The predictions may be made for times corresponding to the times that the candidate medicament doses are given, or may be for more, fewer, or different times. Consequently, a set of predicted analyte values may be generated for each set of proposed medicament doses.

A set of predicted analyte values may be represented as an array or vector of size P. For instance, an analyte vector may be represented as $A_p$, where $A_p(i)$ represents the $i$th analyte prediction in the set and $0 < i \leq P$. For a given set M of medicament doses, $A_p(i)$ may represent the predicted analyte value at the time that dose $M_p(i)$ is given.

According to some examples, the method includes providing each set of analyte predictions and the corresponding proposed medicament doses to a cost function at block 1012.

As discussed above in connection with FIG. 3A - FIG. 3C, an AMD system may apply an MPC algorithm. The MPC algorithm may utilize a cost function that weighs excursions of predicted analyte and medicament trajectories against the target analyte concentrations across a wide range of possible predictions. The MPC algorithm then determines the trajectory with the lowest cost and selects the associated medicament value as the optimum solution for recommended medicament delivery. This cost function may be represented as follows for an AMD measuring glucose as an analyte and providing insulin as a medicament:

$$J(i) = \sum_{j=1}^{P} Q(G_p(j) - SP(j))^2 + R(I_p(j) - B(j))^2$$

The glucose and inulin values may of course be swapped for different analyte and medicament values as appropriate.

Here, the predicted glucose G(j) and insulin I(j) trajectories are compared against the target glucose concentration SP(j) and basal values B(j), respectively, over j cycles (e.g., 5-minute cycles), up to a prediction horizon of P cycles. As described above, the predicted glucose G(j) and/or insulin I(j) trajectories may be based on models of the user's (or a population's) blood-glucose response given small doses of insulin.

The relative weighting of the glucose and insulin excursions are represented as Q and R, respectively. However, this representation applies an equal contribution of excursions across all predicted cycles from the 1st predicted cycle to the Pth predicted cycle to the total cost, J. In other words, the weightings Q and R do not change from cycle-to-cycle up to the prediction horizon.

According to some examples, the method includes weighting each analyte prediction / proposed medicament dose based on time into prediction horizon at block 1014.

In exemplary embodiments, the cost function may be modified to apply a variable weight to the glucose and insulin excursions, depending on the length of the predicted cycles (greater length refers to a point in time further in the future relative to the present), given that the reliability and accuracy of the predicted trajectories may be reduced for cycles further into the predictions (i.e., closer to the end of the prediction horizon P). For purposes of illustration, one example of such a cost function can be represented as the following:

$$J(i) = Q \sum_{j=1}^{M} W_G(j)(G_p(j) - SP(j))^2 + R \sum_{j=1}^{N} W_I(j)(I_p(j) - B(j))^2$$

Here, the analyte (glucose) and medicament (insulin) excursions are separated into individual prediction horizons M and N, with their individual overall weights Q and R. Further, a variable weight for glucose $W_g(i)$ and $W_l(i)$ are represented as a function of the number of cycles of prediction j.

In some embodiments, the weights may be changed to a predetermined or fixed value depending on how far into the prediction horizon the function is evaluating. One example of such predetermined, fixed values may be:

$$W_G(j) = \begin{cases} 1 & j = 1,2,\ldots,M \\ 0 & j > M \end{cases}$$

$$W_I(j) = \begin{cases} 1 & j = 1,2,\ldots,N \\ 0 & j > N \end{cases}$$

In this example, the predetermined, fixed value of 1 is applied until j reaches a predetermined threshold value (M in the case of G(j) and N in the case of I(j)). After this threshold value (also referred to as threshold cycle) is reached the weight is set to a second predetermined, fixed value of 0. The specific predetermined, fixed values selected may be modified based on the application and/or context.

In some embodiments, the weights may be set to predetermined fixed values that vary with the value of j. For example, a weight that results in diminishing a coefficient with increasing values of j (representing cycles further into the prediction horizons) may be applied. For example, the weights may exponentially decrease, e.g. by 50% per cycle as in the following example:

$$W_G(j) = \begin{cases} 1, \frac{1}{2}, \frac{1}{4}, \dots \frac{1}{2^{j-1}} & j = 1,2,\dots,M \\ 0 & j > M \end{cases}$$

$$W_I(j) = \begin{cases} 1, \frac{1}{2}, \frac{1}{4}, \dots \frac{1}{2^{j-1}} & j = 1,2,\dots,N \\ 0 & j > N \end{cases}$$

Alternately, this weight may simply be a monotonically decreasing weight by an integer value in the denominator, as follows:

$$W_G(j) = \begin{cases} 1, \frac{1}{2}, \frac{1}{3}, \dots \frac{1}{j} & j = 1,2,\dots,M \\ 0 & j > M \end{cases}$$

$$W_I(j) = \begin{cases} 1, \frac{1}{2}, \frac{1}{3}, \dots \frac{1}{j} & j = 1,2,\dots,N \\ 0 & j > N \end{cases}$$

These are only two examples of possible functions for the diminishing weights applied to the glucose and insulin excursions, and a wide range of functions may be applied depending on the application.

Moreover, rather than a mathematical, fixed function of decreasing weights, a dynamic formulation (a "dynamic formulation" refers to a mathematical or algorithmic adjustment of a parameter over time, based on one or more time-varying inputs) of the weights may be applied based on the difference between the predicted trajectory in the previous cycles, and the actual glucose concentration in the previous cycles, as follows:

$$W_G(j)' = \begin{cases} \min\left( \dfrac{0.1G(i)}{G_{p,i-j}(j) - G(i)}, 1 \right) & j = 1,2,\dots,M \\ 0 & j > M \end{cases}$$

$$W_G(j) = \min\left( W_G(j)', W_G(j-1) \right)$$

Here, the $j^{th}$ step-ahead prediction in the $i$-$j^{th}$ cycle (j cycles prior to the current cycle) is compared against the actual glucose reading in the current cycle, to determine the relative accuracy of the predictability of the current model. Then, this difference is applied against an expected maximum acceptable error in predictability of 10% of the reading (exemplary value), with the weights proportionally decreased if the differences exceeds this maximum acceptable threshold. A limit is applied to ensure weighting of the next step ahead predictions are never greater than the previous prediction step's weighting.

By way of example, consider calculating the weighting for the $4^{th}$ prediction cycle, i.e. j=4, and the current glucose concentration reading G(j)=120 mg/dL. Further, assume that the $4^{th}$ prediction cycle from the predictions 4 cycles ago was 140 mg/dL, i.e. $G_{p,i-4}(4)$=140 mg/dL. In other words, 4 cycles ago (20 minutes ago if 5-minute cycles are used), the algorithm predicted that the user's glucose concentration would be 120 mg/dL at the current time. The actual current reading from the analyte sensor 110 is 140 mg/dL:

$$j = 4$$

$$G(i) = 120$$

$$G_{p,i-4}(4) = 140$$

In this example, the difference in the most recent 4th cycle ahead prediction vs actual reading was 140-120 mg/dL, or 20 mg/dL. This can be compared against 10% of the accurate glucose reading, or 0.1*G(i)=0.1*120=12 mg/dL. Therefore, the proposed weighting for the 4th prediction cycle $W_G(4)'$=12/20, or 3/5. This can be expressed in numerical form as follows:

$$W_G(4)' = \begin{cases} \min\left(\frac{0.1 \bullet 120}{140-120}, 1\right) & j = 1, 2, \ldots, M \\ 0 & j > M \end{cases}$$

$$= \frac{12}{20}$$

Note that the equation $W_G(j) = min(W_G(j)', W_G(j-1))$ ensures that the weighting for each subsequent $j$th prediction cycle should not exceed the previous J-1th cycle's weighting, and thus in this numerical example, if $W_G(3)$ was lower than 3/5, such as 1/3, then $W_G(4)$ would also be at most 1/3.

The variable weighting based on prediction accuracy as described above applies to analyte (e.g., glucose) trajectories, or $W_G(j)$ in the above formulas. The weighting for the medicament (e.g., insulin) trajectories, or $W_I(j)$ in the above formulas, may be set based on any of the previously described methods, and may be associated with accuracy of the analyte trajectories or independently based on actual medicament delivery trajectories.

In some embodiments (which may be used separately or in conjunction with the preceding embodiments), the prediction horizon may be dynamically adjusted based on past and current variability of analyte readings. This can reduce the risk of erroneous medicament delivery during periods of high analyte variability, and improve responses during periods of consistent, high risk analyte trends. Essentially, analyte predictions tend to be more accurate for shorter prediction horizons than longer ones. If the horizon is shortened, the estimated analyte values (predicted into the future) will typically be more accurate.

An AMD system may predict analyte values into the future. For example, when the analyte is blood glucose concentration, the AMD system may apply a model of insulin and glucose interactions, such as follows:

$$G(i) = b_1 G(i-1) + b_2 G(i-2) + b_3 G(i-3) \ldots + KI(i-3) + \cdots$$

Here, the current glucose concentration G(i) may be a weighted sum (governed by the values of the coefficients $b_i$, $b_2$, $b_3$...) applied on a number of previous glucose values (such as 3 glucose values) and insulin delivery values I(i).

Such models may be utilized to predict a future trajectory based on a set Prediction horizon *P,* by iterating the previously predicted values as the input for the next predicted values. For example, the next predicted glucose concentration from the first equation may be calculated as follows:

$$G(i+1) = b_1 G(i) + b_2 G(i-1) + b_3 G(i-2) \ldots + KI(i-2) + \cdots$$
$$\vdots$$
$$G(i+P) = b_1 G(i+P-1) + b_2 G(i+P-2) + b_3 G(i+P-3) \ldots + KI(i+P-3) + \cdots$$

An AMD system may calculate a wide range of such predictions over P cycles, and calculate a cost to each prediction. An exemplary cost function has been previously described.

Note that the above-described cost function, *J,* may penalize a deviation between the predicted glucose and insulin trajectories G(j) and I(j) against the target glucose SP(j), and basal profile B(j), respectively, at the current $i$th cycle, over the *P* prediction horizon.

However, in conventional systems, the prediction horizon P is a fixed value regardless of prior glucose trajectories. As a result, in cases where the proposed model of glucose-insulin dynamics is insufficient to capture the current system behaviors, this may lead to inaccurate predictions and thus sub-optimal insulin delivery.

According to one example, an algorithm may review the glucose variability/noise in the previous N cycles and reduce the prediction horizon P when the variability rises above acceptable levels. For instance, the prediction horizon P may be reduced according to a relationship with the variability, or by fixed amounts when the variability exceeds certain thresholds. In one example, the prediction horizon P may be adjusted according to the following equation:

$$P_N(i) = \min\left(1.2, \max\left(0.5, \frac{400}{\sum_{k=1}^{6}\left(G(i-k) - G(i-k-1)\right)^2}\right)\right)P_o$$

In this example, the summed square of differences in analyte readings at each cycle, over the last 6 cycles is compared against a typical acceptable threshold. 6, in this case, is a tunable parameter; in some cases the prediction horizon $P$ may be a value between 1 and 14, preferably between 3 and 12, more preferably between 5 and 7; further the prediction horizon $P$ may be rounded to the nearest whole number so that the value of P is not a decimal), In the above example, the threshold is 400 mg$^2$/dl$^2$, although this value may be adjusted depending on the medicament, analyte, user, and application according to techniques that will be well-known to one of ordinary skill in the art. Here, the "400" represents the square of 20, indicating that if the variation in analyte (e.g., blood glucose) levels is greater than 20 in the past 6 cycles, then the denominator of the equation will be different from the numerator, and the prediction horizon P will be reduced (and vice versa).

The prediction horizon may be proportionally reduced if the differences in analyte readings at each cycle is larger than the threshold, and increased if the differences are smaller than a threshold. The degree of adjustment may be bound by design, such as +20% (upper threshold) and -50% (lower threshold), of the original prediction horizon (although these parameters are also tunable depending on the application, medicament, analyte, user, etc.).

In further examples, more advanced noise characterizations may be employed, such as a Fast Fourier Transform (FFT) and other methods, to quantify the acceptable noise of the system. With an FFT, any values exhibiting a high "frequency" will be considered noise and discarded.

In still further examples, the prediction horizon may be adjusted based on the consistency of change. This may be captured as follows:

$$P_N(i) = \min\left(1.2, \max\left(0.5, \frac{5}{\frac{1}{6}\sum_{k=1}^{6}\left(G(i-k) - G(i-k-1)\right) - \left(G(i-k-1) - G(i-k-2)\right)}\right)\right)P_o$$

In this example, the average rate of change of the differences in the acceleration of analyte readings over the previous 6 cycles (tunable) may be compared against a threshold, such as 5 mg$^2$/dl$^2$ (also tunable).

Although this example has been described in connection with glucose and insulin interactions, with glucose concentration as the analyte measurement and insulin as the medicament, embodiments are not limited to this application. Instead, the prediction horizon can be dynamically adjusted in applications in which any type of analyte reading is capable of exhibiting variability in the manner discussed above for glucose concentration.

According to some examples, the method includes optimizing the cost function to select a set of proposed medicament doses at block 1016. For example, each of the proposed medicament dose set / corresponding analyte prediction set pairs may be submitted to the cost function to generate a cost. The set of proposed medicament doses that resulted in the lowest value for the cost function may be selected. In some embodiments, proposed medicament doses may be evaluated until the value of the cost function falls below a predetermined threshold value, at which point the last evaluated set of proposed medicament doses may be selected.

According to some examples, the method includes controlling the AMD based on next proposed medicament dose(s) at block 1018. At the next time that a dose of medicament is intended to be delivered (e.g., based on a basal dosing cycle), the next proposed medicament dose may be read from the proposed medicament set that was selected at block 1016. The algorithm may generate a control signal configured to update the next dosage value in the memory of the control device 102 and/or automated medicament delivery device 108. The control signal may be transmitted to the control device 102 and/or automated medicament delivery device 108, e.g. through wired or wireless communication, over a signal bus, etc. The control signal may be configured to cause the AMD system to deliver medicament at a dosage defined by the dose value. For example, the control signal may be configured to cause a drive mechanism (such as a lead screw connected to a plunger or other suitable mechanism) of the medicament pump 122 to be driven by an amount commensurate with and based on the dose value.

Next, the method may return to block 1006, where a new proposed set of medicament doses may be generated.

The system may repeat the method to generate new proposed medicament doses at predetermined intervals, such as every five minutes, once per hour, etc. In some embodiments, the weights for the cost function may be changed or updated at every iteration through the method, after a predetermined number of iterations, or at predetermined intervals (such as once per hour). The weights may be updated as analyte observations are made and compared to the predicted analyte values generated by the model for a corresponding time period.

If the predictions prove to be relatively accurate (e.g., accurate to within a predetermined threshold amount, such as 10%), the weight for the predictions in that time period may be increased. If they prove to be relatively inaccurate (e.g., not accurate to within the predetermined threshold amount, or not accurate to a second predetermined threshold amount greater than the first predetermined threshold amount), then the weight may be decreased. For example, if it is determined that predictions out to the 4th cycle are relatively accurate but accuracy diminishes beyond the 4th cycle, then the weights for the predictions for cycles where $i > 4$ may be decreased. If, after a period of operation, the model is updated to better capture the user's medicament/analyte dynamics resulting in better predictions out to the 6th cycle, then the weights for early cycles (up to and including the 6th cycle, or the weights for the 5th and 6th cycles that were previously decreased) may be increased.

The components and features of the devices described above may be implemented using any combination of discrete circuitry, application specific integrated circuits (ASICs), logic gates and/or single chip architectures. Further, the features of the devices may be implemented using microcontrollers, programmable logic arrays and/or microprocessors or any combination of the foregoing where suitably appropriate. It is noted that hardware, firmware and/or software elements may be collectively or individually referred to herein as "logic" or "circuit."

It will be appreciated that the exemplary devices shown in the block diagrams described above may represent one functionally descriptive example of many potential implementations. Accordingly, division, omission or inclusion of block functions depicted in the accompanying figures does not infer that the hardware components, circuits, software and/or elements for implementing these functions would be necessarily be divided, omitted, or included in embodiments.

At least one computer-readable storage medium may include instructions that, when executed, cause a system to perform any of the computer-implemented methods described herein.

Some embodiments may be described using the expression "one embodiment" or "an embodiment" along with their derivatives. These terms mean that a particular feature, structure, or characteristic described in connection with the embodiment is included in at least one embodiment. The appearances of the phrase "in one embodiment" in various places in the specification are not necessarily all referring to the same embodiment. Moreover, unless otherwise noted the features described above are recognized to be usable together in any combination. Thus, any features discussed separately may be employed in combination with each other unless it is noted that the features are incompatible with each other.

With general reference to notations and nomenclature used herein, the detailed descriptions herein may be presented in terms of program procedures executed on a computer or network of computers. These procedural descriptions and representations are used by those skilled in the art to most effectively convey the substance of their work to others skilled in the art.

A procedure is here, and generally, conceived to be a self-consistent sequence of operations leading to a desired result. These operations are those requiring physical manipulations of physical quantities. Usually, though not necessarily, these quantities take the form of electrical, magnetic or optical signals capable of being stored, transferred, combined, compared, and otherwise manipulated. It proves convenient at times, principally for reasons of common usage, to refer to these signals as bits, values, elements, symbols, characters, terms, numbers, or the like. It should be noted, however, that all of these and similar terms are to be associated with the appropriate physical quantities and are merely convenient labels applied to those quantities.

Further, the manipulations performed are often referred to in terms, such as adding or comparing, which are commonly associated with mental operations performed by a human operator. No such capability of a human operator is necessary, or desirable in most cases, in any of the operations described herein, which form part of one or more embodiments. Rather, the operations are machine operations. Useful machines for performing operations of various embodiments include general purpose digital computers or similar devices.

Some embodiments may be described using the expression "coupled" and "connected" along with their derivatives. These terms are not necessarily intended as synonyms for each other. For example, some embodiments may be described using the terms "connected" and/or "coupled" to indicate that two or more elements are in direct physical or electrical contact with each other. The term "coupled," however, may also mean that two or more elements are not in direct contact with each other, but yet still co-operate or interact with each other.

Various embodiments also relate to apparatus or systems for performing these operations. This apparatus may be specially constructed for the required purpose or it may comprise a general purpose computer as selectively activated or reconfigured by a computer program stored in the computer. The procedures presented herein are not inherently related to a particular computer or other apparatus. Various general purpose machines may be used with programs written in accordance with the teachings herein, or it may prove convenient to construct more specialized apparatus to perform the required method steps. The required structure for a variety of these machines will appear from the description given.

It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, it can be seen that various features are grouped together in a single embodiment for the purpose of streamlining the disclosure. This method of disclosure is not to be interpreted as reflecting an intention that the claimed embodiments require more features than are expressly recited in each claim. Rather, as the following claims reflect, inventive subject matter lies in less than all features of a single disclosed embodiment. Thus the following claims are hereby incorporated into the Detailed Description, with each claim standing on its own as a separate embodiment. In the appended claims, the terms "including" and "in which" are used as the plain-English equivalents of the respective terms "comprising" and "wherein," respectively. Moreover, the terms "first," "second," "third," and so forth, are used merely as labels, and are not intended to impose numerical requirements on their objects.

What has been described above includes examples of the disclosed architecture. It is, of course, not possible to describe every conceivable combination of components and/or methodologies, but one of ordinary skill in the art may recognize that many further combinations and permutations are possible. Accordingly, the novel architecture is intended to embrace all such alterations, modifications and variations that fall within the spirit and scope of the appended claims.

The components and features of the devices described above may be implemented using any combination of discrete circuitry, application specific integrated circuits (ASICs), logic gates and/or single chip architectures. Further, the features of the devices may be implemented using microcontrollers, programmable logic arrays and/or microprocessors or any combination of the foregoing where suitably appropriate. It is noted that hardware, firmware and/or software elements may be collectively or individually referred to herein as "logic" or "circuit."

It will be appreciated that the exemplary devices shown in the block diagrams described above may represent one functionally descriptive example of many potential implementations. Accordingly, division, omission or inclusion of block functions depicted in the accompanying figures does not infer that the hardware components, circuits, software and/or elements for implementing these functions would necessarily be divided, omitted, or included in embodiments.

At least one computer-readable storage medium may include instructions that, when executed, cause a system to perform any of the computer-implemented methods described herein.

Some embodiments may be described using the expression "one embodiment" or "an embodiment" along with their derivatives. These terms mean that a particular feature, structure, or characteristic described in connection with the embodiment is included in at least one embodiment. The appearances of the phrase "in one embodiment" in various places in the specification are not necessarily all referring to the same embodiment. Moreover, unless otherwise noted the features described above are recognized to be usable together in any combination. Thus, any features discussed separately may be employed in combination with each other unless it is noted that the features are incompatible with each other.

With general reference to notations and nomenclature used herein, the detailed descriptions herein may be presented in terms of program procedures executed on a computer or network of computers. These procedural descriptions and representations are used by those skilled in the art to most effectively convey the substance of their work to others skilled in the art.

A procedure is here, and generally, conceived to be a self-consistent sequence of operations leading to a desired result. These operations are those requiring physical manipulations of physical quantities. Usually, though not necessarily, these quantities take the form of electrical, magnetic or optical signals capable of being stored, transferred, combined, compared, and otherwise manipulated. It proves convenient at times, principally for reasons of common usage, to refer to these signals as bits, values, elements, symbols, characters, terms, numbers, or the like. It should be noted, however, that all of these and similar terms are to be associated with the appropriate physical quantities and are merely convenient labels applied to those quantities.

Further, the manipulations performed are often referred to in terms, such as adding or comparing, which are commonly associated with mental operations performed by a human operator. No such capability of a human operator is necessary, or desirable in most cases, in any of the operations described herein, which form part of one or more embodiments. Rather, the operations are machine operations. Useful machines for performing operations of various embodiments include general purpose digital computers or similar devices.

Some embodiments may be described using the expression "coupled" and "connected" along with their derivatives. These terms are not necessarily intended as synonyms for each other. For example, some embodiments may be described using the terms "connected" and/or "coupled" to indicate that two or more elements are in direct physical or electrical contact with each other. The term "coupled," however, may also mean that two or more elements are not in direct contact with each other, but yet still co-operate or interact with each other.

Various embodiments also relate to apparatus or systems for performing these operations. This apparatus may be specially constructed for the required purpose or it may comprise a general purpose computer as selectively activated or reconfigured by a computer program stored in the computer. The procedures presented herein are not inherently related to a particular computer or other apparatus. Various general purpose machines may be used with programs written in accordance with the teachings herein, or it may prove convenient to construct more specialized apparatus to perform the required method steps. The required structure for a variety of these machines will appear from the description given.

It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, it can be seen that various features are grouped together in a single embodiment for the purpose of streamlining the disclosure. This method of disclosure is not to be interpreted as reflecting an intention that the claimed embodiments require more features than are expressly recited in each claim. Rather, as the following claims reflect, inventive subject matter lies in less than all features of a single disclosed embodiment. Thus the following claims are hereby incorporated into the Detailed Description, with each claim standing on its own as a separate embodiment. In the appended claims, the terms "including" and "in which" are used as the plain-English equivalents of the respective terms "comprising" and "wherein," respectively. Moreover, the terms "first," "second," "third," and so forth, are used merely as labels, and are not intended to impose numerical requirements on their objects.

What has been described above includes examples of the disclosed architecture. It is, of course, not possible to describe every conceivable combination of components and/or methodologies, but one of ordinary skill in the art may recognize that many further combinations and permutations are possible. Accordingly, the novel architecture is intended to embrace all such alterations, modifications and variations that fall within the spirit and scope of the appended claims.

Examples of the present invention may be defined by the following descriptions:

1. A computer-implemented method comprising:

accessing a model of analyte-medicament dynamics associated with a user of an automated medicament delivery device;

generating two or more sets of proposed medicament doses representing medicament doses to be delivered to the user over a period of time defined by a prediction horizon;

for each set of the proposed medicament doses, using the model to calculate a set of predicted analyte values corresponding to the proposed medicament doses;

applying a cost function to each set of the proposed medicament doses and corresponding set of predicted analyte values, wherein the cost function weighs values that represent earlier doses in the sets to a greater degree than values that represent later doses in the sets that are closer to the prediction horizon;

selecting one of the two or more sets of proposed medicament doses based on an output of the cost function; and

transmitting a control signal configured to cause the automated medicament delivery device to deliver the medicament in an amount defined by the selected set of proposed medicament doses.

2. The computer-implemented method of 1, wherein the weighing applies weights that are set to predetermined fixed values.

3. The computer-implemented method of 1 or 2, wherein the weighing applies a set of weights having a first predetermined fixed value until a threshold cycle is reached, and after the threshold cycle is reached each weight applies a second predetermined fixed value.

4. The computer-implemented method of any of 1-3, wherein the weighing applies weights that decrease exponentially to the prediction horizon.

5. The computer-implemented method of any of 1-4, wherein the weighing applies weights that vary with a value of the current predicted cycle being evaluated.

6. The computer-implemented method of any of 1-5, wherein the weighing applies weights that are calculated according to a dynamic formulation based on a difference between a predicted trajectory in a previous cycle and a measured analyte value in the previous cycle.

7. The computer-implemented method of any of 1-6, wherein the weighing applies one or more weights, and further comprising updating the weights at predetermined intervals.

8. The computer-implemented method of any of 1-7, wherein the model is updated based on a prediction accuracy at predetermined intervals.

9. The computer-implemented method of any of 1-8, wherein the prediction horizon varies based on a prior analyte trajectory.

10. The computer-implemented method of any of 1-9, wherein the prediction horizon is decreased as prior analyte readings exhibit increased variability.

11. A non-transitory computer-readable medium storing instructions that, when executed by one or more processors, cause the processors to perform any of the methods of 1-10.

12. An automated medicament delivery system comprising:

    one or more processors; and

    a non-transitory computer-readable medium storing instructions that, when executed the one or more processors, cause the processors to perform any of the methods of 1-10.

**Claims**

1.  A computer-implemented method comprising:

    accessing a model of analyte-medicament dynamics associated with a user of an automated medicament delivery device;
    generating two or more sets of proposed medicament doses representing medicament doses to be delivered to the user over a period of time defined by a prediction horizon;
    for each set of the proposed medicament doses, using the model to calculate a set of predicted analyte values corresponding to the proposed medicament doses;
    applying a cost function to each set of the proposed medicament doses and corresponding set of predicted analyte values, wherein the cost function weighs values that represent earlier doses in the sets to a greater degree than values that represent later doses in the sets that are closer to the prediction horizon;
    selecting one of the two or more sets of proposed medicament doses, based on an output of the cost function, as the medicament doses to be delivered to the user.

2.  The computer-implemented method of claim 1, wherein the weighing applies weights that are set to predetermined fixed values, or wherein the weighing applies a set of weights having a first predetermined fixed value until a threshold cycle is reached, and after the threshold cycle is reached each weight applies a second predetermined fixed value.

3.  The computer-implemented method of any of claims 1 or 2, wherein the weighing applies weights that decrease exponentially to the prediction horizon, or wherein the weighing applies weights that vary with a value of the current predicted cycle being evaluated.

4.  The computer-implemented method of any of claims 1-3, wherein the weighing applies weights that are calculated according to a dynamic formulation based on a difference between a predicted trajectory in a previous cycle and a measured analyte value in the previous cycle, or wherein the weighing applies one or more weights, and further comprising updating the weights at predetermined intervals.

5.  The computer-implemented method of any of claims 1-4, wherein the model is updated based on a prediction accuracy at predetermined intervals.

6.  The computer-implemented method of any of claims 1-5, wherein the prediction horizon varies based on a prior analyte trajectory, or wherein the prediction horizon is decreased as prior analyte readings exhibit increased variability.

7.  A non-transitory computer-readable medium storing instructions that, when executed by one or more processors, cause the processors to:

access a model of analyte-medicament dynamics associated with a user of an automated medicament delivery device;

generate two or more sets of proposed medicament doses representing medicament doses to be delivered to the user over a period of time defined by a prediction horizon;

for each set of the proposed medicament doses, use the model to calculate a set of predicted analyte values corresponding to the proposed medicament doses;

apply a cost function to each set of the proposed medicament doses and corresponding set of predicted analyte values, wherein the cost function weighs values that represent earlier doses in the sets to a greater degree than values that represent later doses in the sets that are closer to the prediction horizon;

select one of the two or more sets of proposed medicament doses based on an output of the cost function; and

transmit a control signal configured to cause the automated medicament delivery device to deliver the medicament in an amount defined by the selected set of proposed medicament doses.

8. The medium of claim 7, wherein the weighing applies weights that are set to predetermined fixed values.

9. The medium of claim 7 or 8, wherein the weighing applies weights that decrease exponentially to the prediction horizon, or that vary with a value of the current predicted cycle being evaluated.

10. The medium of any of claims 7-9, wherein the model is updated based on a prediction accuracy at predetermined intervals.

11. The medium of any of claims 7-10, wherein the prediction horizon varies based on a prior analyte trajectory.

12. An automated medicament delivery system comprising:

one or more processors; and
a non-transitory computer-readable medium storing instructions that, when executed the one or more processors, cause the processors to

access a model of analyte-medicament dynamics associated with a user of an automated medicament delivery device;

generate two or more sets of proposed medicament doses representing medicament doses to be delivered to the user over a period of time defined by a prediction horizon;

for each set of the proposed medicament doses, use the model to calculate a set of predicted analyte values corresponding to the proposed medicament doses;

apply a cost function to each set of the proposed medicament doses and corresponding set of predicted analyte values, wherein the cost function weighs values that represent earlier doses in the sets to a greater degree than values that represent later doses in the sets that are closer to the prediction horizon;

select one of the two or more sets of proposed medicament doses based on an output of the cost function; and

transmit a control signal configured to cause the automated medicament delivery device to deliver the medicament in an amount defined by the selected set of proposed medicament doses.

13. The system of claim 12, wherein the weighing applies weights that are set to predetermined fixed values.

14. The system of claim 12 or 13, wherein the weighing applies weights that decrease exponentially to the prediction horizon, or that vary with a value of the current predicted cycle being evaluated.

15. The system of any of claims 12-14, wherein the model is updated based on a prediction accuracy at predetermined intervals, or wherein the prediction horizon varies based on a prior analyte trajectory.

**FIG. 1**

EP 4 679 442 A1

FIG. 2A

FIG. 2B

302

**FIG. 3A**

110

**FIG. 3B**

110

112

**FIG. 3C**

FIG. 4

500

DEPLOY ANALYTE SENSOR 502

ANALYTE SENSOR BROADCASTS WIRELESS SIGNAL 504

AMDD STARTUP 506

AMDD RECEIVES WIRELESS SIGNAL 508

DISPLAY PAIRING INFORMATION 510

REQUEST USER CONFIRMATION 512

AUTOMATICALLY PAIR ANALYTE SENSOR 514

REMOVE EXISTING ANALYTE SENSOR 524

AMDD AUTOMATICALLY PAIRS WITH REPLACEMENT PUMP 522

AMDD DETECTS WIRELESS SIGNAL 520

REPLACEMENT PUMP BROADCASTS WIRELESS SIGNAL 518

REMOVE EXISTING NON-DURABLE PUMP 516

**FIG. 5**

600

```
┌─────────────────────────────┐
│   ESTIMATE TDM NEEDS BASED ON │
│   POPULATION-BASED DEFAULT    │
│        VALUES 602             │
└─────────────────────────────┘
              │
              ▼
┌ ─ ─ ─ ─ ─ ─ ─ ─ ─ ─ ─ ─ ─ ─ ┐
    RECEIVE USER-SPECIFIED INITIAL
│        TDM VALUE 604          │
└ ─ ─ ─ ─ ─ ─ ─ ─ ─ ─ ─ ─ ─ ─ ┘
              │
              ▼
┌ ─ ─ ─ ─ ─ ─ ─ ─ ─ ─ ─ ─ ─ ─ ┐
│  CALCULATE WEIGHTED TDM VALUE │
              606
└ ─ ─ ─ ─ ─ ─ ─ ─ ─ ─ ─ ─ ─ ─ ┘
              │
              ▼
┌─────────────────────────────┐
│   ESTABLISH INITIAL SAFE BOLUS│
│  LIMIT BASED ON (WEIGHTED) TDM│
│             608               │
└─────────────────────────────┘
```

**FIG. 6**

700

SET INITIAL MOB CONSTRAINT
702

SET INITIAL MAXIMUM CYCLE DOSE
704

ADJUST UPPER BOUNDS BASED ON
USER-INPUT TDM 706

DETERMINE NEXT DOSE AMOUNT
708

EXCEEDS SAFETY
CONSTRAINT(S)? 710 — YES → DELIVER CONSTRAINED AMOUNT
712

NO

DELIVER DETERMINED DOSE 714

**FIG. 7A**

**FIG. 7B**

EP 4 679 442 A1

```
            ┌──────────────────────┐
            │  PRESENT ALERT 730   │
            └──────────┬───────────┘
                       │
                       ▼
            ┌──────────────────────┐
            │   START TIMER 732    │
            └──────────┬───────────┘
                       │
                       ▼
                  ╱─────────╲
         ╱───────╱ USER INPUT ╲───────── YES, LESS THAN THRESHOLD
         │       ╲ RECEIVED? 734 ╱                    │
         │        ╲─────────╱                         │
         │             │                              │
         NO            │ YES, GREATER THAN THRESHOLD  │
         │             ▼                              ▼
         │   ┌──────────────────────┐    ┌──────────────────────┐
         └──▶│ MAINTAIN OR INCREASE │    │ DECREASE FREQUENCY 738│
             │   ALERT FREQUENCY 736│    │                      │
             └──────────────────────┘    └──────────────────────┘
```

**FIG. 7C**

800

START TIMER AT INITIALIZATION
802

EMERGENCY
THRESHOLD? 804 —YES→ ADAPT 814

NO

EXECUTE ONE OR MORE
MEDICAMENT DELIVERY CYCLES
806

REDUCE ADAPTATION THRESHOLD BASED ON
CURRENT AND HISTORICAL ANALYTE 808

ADJUST ADAPTATION THRESHOLD BASED ON
DIFFERENCE BETWEEN MANUAL AND
DEFAULT TDM 810

ADAPTATION THRESHOLD
REACHED? 812

YES

**FIG. 8**

900

FIG. 9

START <u>1002</u>

ACCESS A MODEL OF USER'S ANALYTE-MEDICAMENT DYNAMICS <u>1004</u>

GENERATE TWO OR MORE SETS OF PROPOSED MEDICAMENT DOSES <u>1006</u>

CONTROL AMD BASED ON NEXT PROPOSED MEDICAMENT DOSE(S) <u>1018</u>

PROVIDE PROPOSED MEDICAMENT DOSE SETS TO MODEL <u>1008</u>

OPTIMIZE COST FUNCTION TO SELECT A SET OF PROPOSED MEDICAMENT DOSES <u>1016</u>

GENERATE ANALYTE PREDICTIONS FOR EACH PROPOSED MEDICAMENT DOSE SET <u>1010</u>

PROVIDE EACH SET OF ANALYTE PREDICTIONS AND THE CORRESPONDING PROPOSED MEDICAMENT DOSES TO A COST FUNCTION <u>1012</u>

WEIGHT EACH ANALYTE PREDICTION / PROPOSED MEDICAMENT DOSE BASED ON TIME INTO PREDICTION HORIZON <u>1014</u>

**FIG. 10**

**EUROPEAN SEARCH REPORT**

Application Number

EP 25 18 7914

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2024/066224 A1 (ZHENG YIBIN [US] ET AL) 29 February 2024 (2024-02-29) * paragraphs 5-8, 55-62, 47 figures 8, 9, 7A, * ----- | 1-15 | INV. G16H20/17 G16H40/63 G16H50/20 G16H50/70 |
| Y | WO 2020/060568 A1 (MEDTRONIC MINIMED INC [US]) 26 March 2020 (2020-03-26) * paragraph 157; figure 17 * ----- | 1-15 | |

TECHNICAL FIELDS
SEARCHED      (IPC)

G16H

The present search report has been drawn up for all claims

| Place of search | Date of completion of the search | Examiner |
|---|---|---|
| Munich | 20 November 2025 | Wittke, Claudia |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
.........................................................................................
& : member of the same patent family, corresponding
   document

EPO FORM 1503 03.82 (P04C01)

EP 4 679 442 A1

ANNEX TO THE EUROPEAN SEARCH REPORT
ON EUROPEAN PATENT APPLICATION NO.

EP 25 18 7914

This annex lists the patent family members relating to the patent documents cited in the above-mentioned European search report.
The members are as contained in the European Patent Office EDP file on
The European Patent Office is in no way liable for these particulars which are merely given for the purpose of information.

20-11-2025

| Patent document cited in search report | Publication date | Patent family member(s) | Publication date |
|---|---|---|---|
| US 2024066224 A1 | 29-02-2024 | AU 2023333103 A1 | 06-03-2025 |
| | | EP 4581637 A1 | 09-07-2025 |
| | | JP 2025529149 A | 04-09-2025 |
| | | US 2024066224 A1 | 29-02-2024 |
| | | WO 2024049655 A1 | 07-03-2024 |
| WO 2020060568 A1 | 26-03-2020 | CA 3109754 A1 | 26-03-2020 |
| | | CN 112714936 A | 27-04-2021 |
| | | EP 3853860 A1 | 28-07-2021 |
| | | WO 2020060568 A1 | 26-03-2020 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63668620